(19) Europäisches Patentamt
European Patent Office
Office européen des brevets

(11) **EP 2 944 952 B1**

(12) **EUROPÄISCHE PATENTSCHRIFT**

(45) Veröffentlichungstag und Bekanntmachung des Hinweises auf die Patenterteilung:
**11.01.2023 Patentblatt 2023/02**

(21) Anmeldenummer: **15167592.3**

(22) Anmeldetag: **13.05.2015**

(51) Internationale Patentklassifikation (IPC):
**G01N 27/60** *(2006.01)* **G01N 27/447** *(2006.01)*

(52) Gemeinsame Patentklassifikation (CPC):
**G01N 27/60;** G01N 27/44752

(54) **SYSTEM ZUM ERMITTELN DES ZETAPOTENZIALS ZUM CHARAKTERISIEREN EINER FEST-FLÜSSIG-PHASENGRENZE MIT GESTEUERTER DRUCKPROFILBEAUFSCHLAGUNG**

SYSTEM FOR DETERMINING THE ZETA POTENTIAL FOR CHARACTERISING A FIXED LIQUID PHASE BORDER WITH CONTROLLED PRESSURE PROFILE APPLICATION

SYSTÈME DE DÉTERMINATION DU POTENTIEL DE ZÊTA DESTINÉ À CARACTÉRISER UNE LIMITE DE PHASE LIQUIDE/SOLIDE AVEC APPLICATION DE PROFIL DE PRESSION COMMANDÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.05.2014 AT 503362014**

(43) Veröffentlichungstag der Anmeldung:
**18.11.2015 Patentblatt 2015/47**

(73) Patentinhaber: **Anton Paar GmbH 8054 Graz (AT)**

(72) Erfinder: **Luxbacher, Thomas 8045 Graz (AT)**

(74) Vertreter: **Dilg, Haeusler, Schindelmann Patentanwaltsgesellschaft mbH Leonrodstraße 58 80636 München (DE)**

(56) Entgegenhaltungen:
WO-A1-86/00707    WO-A1-2007/065825
DD-A1- 258 470    DE-A1- 10 154 790
DE-U1- 20 209 563    JP-A- S6 247 545
JP-B2- H 063 425    US-A- 6 023 661

• ZIMMERMANN R ET AL: "BESTIMMUNG DES ZETA-POTENTIALS UND DER GRENZFLAECHENLEITFAEHIGKEIT DURCH STROEMUNGSPOTENTIAL- UND STROEMUNGSSTROMMESSUNGEN//DETERMINATION OF THE ZETA POTENTIAL AND THE SURFACE CONDUCTIVITY BY STREAMING POTENTIAL AND STREAMING CURRENT MEASUREMENTS", TECHNISCHES MESSEN TM, R.OLDENBOURG VERLAG. MUNCHEN, DE, Bd. 67, Nr. 9, 1. September 2000 (2000-09-01), Seiten 353-360, XP001132480, ISSN: 0171-8096

## Beschreibung

Die Erfindung betrifft eine Vorrichtung und ein Verfahren zum Ermitteln von für ein Zetapotenzial indikativer Information zum Charakterisieren einer Grenzfläche zwischen einer festen Phase und einer flüssigen Phase.

Das elektrokinetische oder Zetapotenzial $\zeta$ beschreibt die Ladungsverteilung an der Grenzfläche zweier nicht mischbarer Phasen. Bedeutung hat das Zetapotenzial für die Charakterisierung der Grenzfläche fest-flüssig. An der Grenzfläche zwischen einer makroskopischen Materialoberfläche und einer Flüssigkeit kann das Zetapotenzial aus Messungen des Strömungspotenzials und des Strömungsstroms berechnet werden. Materialien mit makroskopischen Oberflächen sind Probenkörper unterschiedlicher Form und Größe zuzuordnen. Dazu zählen Proben mit flacher Oberfläche, Faserproben, Granulat und Pulver mit Partikelgröße größer 1 $\mu$m.

Für die Messung des Strömungspotenzials und Strömungsstroms kann die Festkörperprobe in einer Messzelle derart angeordnet werden, dass eine Kapillare oder ein Kapillarsystem mit geeigneter hydraulischer Permeabilität entsteht. Die Flüssigkeitsströmung durch diese Kapillare (Strömungskanal) erzeugt eine Druckdifferenz und ein elektrisches Signal, das entweder als Spannung (Strömungspotenzial) oder Strom (Strömungsstrom) gemessen wird. Festkörper mit flacher Oberfläche werden beispielsweise parallel zueinander angeordnet und es entsteht eine Kapillare mit rechteckiger Querschnittsfläche. Faserproben und Granulat werden beispielsweise in Pfropfenform angeordnet und das dadurch entstehende, unregelmäßige Kapillarsystem durchströmt.

Die Berechnung des Zetapotenzials erfolgt nach den klassischen Gleichungen nach Helmholtz und von Smoluchowski. Für die Berechnung aus Messungen des Strömungsstroms $I_{str}$ gilt:

$$\zeta = \frac{dI_{str}}{d\Delta p} \cdot \frac{\eta}{\varepsilon \cdot \varepsilon_0} \cdot \frac{L}{A} \qquad \text{(Gleichung 1)}$$

mit $dI_{str}/d\Delta p$ - Strömungsstromkoeffizient (Änderung des Strömungsstroms mit Druckdifferenz über die Länge des Strömungskanals), $\eta$ - dynamische Viskosität der Flüssigkeit, $\varepsilon$ - Dielektrizitätskoeffizient der Flüssigkeit, $\varepsilon_0$ - Permittivität, $L$ - Länge des Strömungskanals, $A$ - Querschnitt des Strömungskanals.

Die Berechnung des Zetapotenzials aus Messungen des Strömungspotenzials $U_{str}$ erfolgt nach:

$$\zeta = \frac{dU_{str}}{d\Delta p} \cdot \frac{\eta}{\varepsilon \cdot \varepsilon_0} \cdot \kappa \qquad \text{(Gleichung 2)}$$

mit $dU_{str}/d\Delta p$ - Strömungspotenzialkoeffizient (Änderung des Strömungspotenzials mit Druckdifferenz über die Länge des Strömungskanals), $\kappa$ - elektrische Leitfähigkeit der Flüssigkeit.

Der Zusammenhang zwischen Zetapotenzial und Strömungsstrom (Gleichung 1) bzw. Strömungspotenzial (Gleichung 2) führt nur dann zu gleichen Ergebnissen, wenn es sich um eine nichtleitende Festkörperprobe handelt. Die Identität der Zetapotenzialwerte ist zudem von der Elektrolytkonzentration abhängig. Besonders bei geringer Ionenstärke ($I$ < 0.001 mol/l) beeinflusst die Oberflächen- oder Grenzflächenleitfähigkeit die korrekte Bestimmung des Zetapotenzials nach Gleichung 2. Das Zetapotenzial leitfähiger Festkörperoberflächen (elektronisch leitfähig, beispielsweise Metalle, oder ionisch leitfähig, beispielsweise poröse Festkörper oder quellfähige Schichten oder Materialien) lässt sich auch bei höherer Ionenstärke ($I \geq 0.001$ mol/l) nach Gleichung 2 nicht korrekt bestimmen. Diese Einschränkungen verlangen die Messung des Strömungsstroms anstelle des Strömungspotenzials und die Berechnung des Zetapotenzials nach Gleichung 1.

Die Messung von Strömungspotenzial und Strömungsstrom erfolgt zum Beispiel mit Messelektroden aus unterschiedlichen Materialien, Größe und Bauart. Elektroden unterliegen dem Vorgang der Polarisation, die verschiedene Ursachen haben kann:

Es werden entweder Elektroden erster Art (beispielsweise Platin-Elektroden) oder Elektroden zweiter Art (reversible Elektroden, beispielsweise Silber-Silberchlorid-Elektroden) verwendet. Polarisationseffekte treten insbesondere bei Elektroden erster Art und weniger ausgeprägt bei Elektroden zweiter Art auf.

Die Elektrodenpolarisation ist auch von der spezifischen Oberfläche der Elektroden abhängig. So wird beispielsweise die Oberfläche von Platinelektroden (Elektroden erster Art) durch elektrochemische Aufbringung einer porösen Platinschicht (platinum black) vergrößert. Die Oberfläche der beispielsweise auf Silberelektroden abgeschiedenen Silberchloridschicht ist ebenfalls porös und verringert dadurch die Neigung zu Elektrodenpolarisation.

Elektrodenpolarisation ist vor allem eine Eigenschaft der Elektrolytkonzentration (Ionenstärke). Polarisationseffekte treten an Elektroden erster Art bereits bei geringer Ionenstärke eines in Wasser gelösten Elektrolyten auf. Je nach Qualität (Größe, Qualität der Beschichtung) und spezifischer Oberfläche nimmt die Elektrodenpolarisation auch bei Elektroden zweiter Art ab einer bestimmten Ionenstärke deutlich zu.

Die Neigung zu Polarisationseffekten von Elektroden hat einen Einfluss sowohl auf die Messung des Strömungsstroms als auch auf die Messung des Strömungspotenzials. Der Einfluss auf die Strommessung ist mitunter größer als jener auf die Spannungsmessung. Bei geringen Messsignalen des Strömungspotenzials und Strömungsstroms erhöht die Elektrodenpolarisation den Fehler der Messung und verringert dadurch die Qualität des nach Gleichung 1 oder Gleichung 2 berechneten Zetapotenzials.

Es gibt eine Reihe an kommerziellen Messgeräten für die Messung des Strömungspotenzials, aber auch des Strömungsstroms zur Bestimmung des Zetapotenzials an makroskopischen Festkörperoberflächen. In diesen Messgeräten wurde dem Effekt der Elektrodenpolarisation, beschrieben durch das Ungleichgewicht in der Potenzialdifferenz zwischen den beiden Messelektroden im Ruhezustand, durch entsprechende bauliche Maßnahmen und Messprotokolle begegnet.

Bei einer Zweipunktmessung werden der Spannungswert im Ruhezustand (keine Flüssigkeitsströmung, Asymmetriepotenzial Uo) und das Strömungspotenzial bei einer konstanten Druckdifferenz, $U_{str}(\Delta p)$, verwendet, um den Strömungspotenzialkoeffizienten in Gleichung 2 als Differenzenquotient $\Delta U_{str}/\Delta p$ mit $\Delta U_{str} = U_{str}(\Delta p)$ - Uo zu berechnen. Diese Methode ist für Messbedingungen geeignet, in denen das Asymmetriepotenzial Uo einen geringen Beitrag zum gemessenen Strömungspotenzial liefert (< 10%).

Bei einer Druckstufenmessung wird das Strömungspotenzial $U_{str}(\Delta p)$ bei unterschiedlichen konstanten Druckdifferenzen bestimmt und der Strömungspotenzialkoeffizient in Gleichung 2 aus der linearen Regression der Messpunkte $U_{str}$ versus $\Delta p$ berechnet. Durch die größere Anzahl an Messpunkten gegenüber der Zweipunktmessung wird die Qualität des berechneten Strömungspotenzialkoeffizienten verbessert.

Die technische Realisierung solcher Methoden zur Bestimmung des Strömungspotenzial- und Strömungsstromkoeffizienten erfolgt in kommerziellen Messgeräten und provisorischen Messaufbauten durch Anlegen einer Druckdifferenz mittels externer Pumpe oder Gasdruck.

Die Messung des Strömungspotenzials und des Strömungsstroms nach den herkömmlichen Messmethoden beschränkt sich allerdings auf die Bestimmung des Zetapotenzials bei geringen Ionenstärken.

Weiterer Stand der Technik ist in WO 86/00707, Pu et al., "Label-free detection of heparin, streptavidin, and other probes by pulsed streaming potenzials in plastic microfluidic channels", Anal Chem 2008 Sep. 1;80(17):6532-6, Luna-Vera, "Adsorption kinetics of proteins in plastic microfluidic channels: Real-time monitoring of lysozyme adsorption by pulsed streaming potenzials", Biosensors and Bioelectronics 25 (2010) 1539-1543, JPH02216443, WO2007/065825, DD258470,DE10154790, JP62047545, DE20209563,JP11190711,JP04050758  und US 6,023,661  offenbart.

Es ist eine Aufgabe der vorliegenden Erfindung, das Ermitteln von für ein Zetapotenzial indikativer Information zur Charakterisierung einer Fest-Flüssig-Probe mit hoher Genauigkeit und insbesondere auch für hohe Ionenstärken zu ermöglichen.

Diese Aufgabe wird durch die Gegenstände mit den Merkmalen gemäß den unabhängigen Patentansprüchen gelöst. Weitere Ausführungsbeispiele sind in den abhängigen Ansprüchen gezeigt.

Gemäß einem Ausführungsbeispiel der vorliegenden Erfindung ist eine Vorrichtung zum Ermitteln von für ein Zetapotenzial indikativer Information zum Charakterisieren einer Grenzfläche zwischen einer festen Phase und einer flüssigen Phase geschaffen, wobei die Vorrichtung einen Druckbehälter, in dem die flüssige Phase aufnehmbar ist, eine Messzelle, die stromabwärts von und in Fluidkommunikation mit dem Druckbehälter bringbar bzw. in Fluidkommunikation mit dem Druckbehälter befindlich angeordnet ist und in der die feste Phase aufnehmbar ist, einen Vorratsbehälter, der stromabwärts von und in Fluidkommunikation mit der Messzelle befindlich bzw. in Fluidkommunikation mit der Messzelle bringbar angeordnet ist, eine Druckbeaufschlagungseinrichtung, die zum Beaufschlagen des Druckbehälters mit einem Druckprofil mit einer zeitlich kontinuierlichen Druckänderung derart eingerichtet ist, dass dadurch flüssige Phase aus dem Druckbehälter durch die Messzelle in den Vorratsbehälter förderbar ist, und eine Erfasseinrichtung zum Erfassen der für das Zetapotenzial indikativen Information an der Messzelle während des Beaufschlagens des Druckbehälters mit dem Druckprofil aufweist.

Gemäß einem weiteren Ausführungsbeispiel der vorliegenden Erfindung ist ein Verfahren zum Ermitteln von für ein Zetapotenzial indikativer Information zum Charakterisieren einer Grenzfläche zwischen einer festen Phase und einer flüssigen Phase bereitgestellt, wobei bei dem Verfahren die flüssige Phase in einem Druckbehälter aufgenommen wird, die feste Phase in einer Messzelle aufgenommen wird, die stromabwärts von und in Fluidkommunikation mit dem Druckbehälter bringbar bzw. in Fluidkommunikation mit dem Druckbehälter befindlich angeordnet ist, ein Vorratsbehälter stromabwärts von und in Fluidkommunikation mit der Messzelle befindlich bzw. in Fluidkommunikation mit der Messzelle bringbar angeordnet wird, der Druckbehälter mit einem Druckprofil mit einer zeitlich kontinuierlichen Druckänderung derart beaufschlagt wird, dass dadurch flüssige Phase aus dem Druckbehälter durch die Messzelle in den Vorratsbehälter förderbar ist, und die für das Zetapotenzial indikative Information an der Messzelle während des Beaufschlagens des Druckbehälters mit dem Druckprofil erfasst wird.

Gemäß einem exemplarischen Ausführungsbeispiel kann zum Charakterisieren einer Grenzfläche zwischen einer festen Phase und einer flüssigen Phase Information über ein zugehöriges Zetapotenzial ermittelt werden, indem während des Durchführens der Messung eine zeitlich kontinuierliche Änderung der Druckdifferenz (insbesondere unter Vorgabe eines sich über die Messung hinweg verändernden Druckgradienten) zwischen dem Druckbehälter und dem Vorratsbehälter eingestellt bzw. steuerungstechnisch vorgegeben wird. Dadurch können zeitabhängige Einflüsse von Elektrodenpolarisation und andere Drifterscheinungen zumindest teilweise kompensiert werden. Dies führt zu einer höheren Genauigkeit und Verlässlichkeit der ermittelten Messergebnisse, auch bei höherer Ionenstärke.

Im Weiteren werden zusätzliche exemplarische Ausführungsbeispiele der Vorrichtung und des Verfahrens beschrieben.

Gemäß einem Ausführungsbeispiel der Erfindung kann die Druckbeaufschlagungseinrichtung ausgebildet sein, der Messzelle ein Druckprofil mit einer pulsationsfreien Druckänderung, insbesondere mit einem monotonen (insbesondere monoton abnehmenden), zum Beispiel linearen Druckverlauf, bereitzustellen. Wenn die sich mit der Zeit ändernde Druckdifferenz zwischen Druckbehälter und Vorratsbehälter ohne Druckpulse oder sonstige Unstetigkeiten im Druck-Zeit-Verlauf ändert, kann die Messung artefaktfrei und somit hochpräzise bzw. reproduzierbar durchgeführt werden.

Gemäß der Erfindung ist die Druckbeaufschlagungseinrichtung eingerichtet, einen Gasraum oberhalb der flüssigen Phase in dem Druckbehälter mit einem komprimierten Gaspolster zu beaufschlagen, um dadurch das Druckprofil mit einer kontinuierlichen Abnahme des Drucks zu erzeugen. Im Inneren des Druckbehälters herrscht ein Überdruck, wobei das Innere des Druckbehälters von der Umgebung druckentkoppelt ist. Anders ausgedrückt wird ein gegenüber der Atmosphäre unter erhöhtem Druck stehendes Gaspolster auf die Flüssigkeitssäule in dem Druckbehälter aufgeprägt. Dieser Druck baut sich kontinuierlich ab, wenn die flüssige Phase aus dem Druckbehälter durch die Messzelle in den Vorratsbehälter fließt, der zum Beispiel auf einem Referenzdruck befindlich (etwa mit Atmosphärendruck verbunden) ist.

Gemäß einem Ausführungsbeispiel der Erfindung kann die Druckbeaufschlagungseinrichtung eine Druckerzeugungseinheit zum Erzeugen von Druck in dem Druckbehälter und ein fluidisches Schaltelement aufweisen, so dass mittels Schaltens des Schaltelements zum fluidischen Koppeln des Druckbehälters mit der Messzelle die Messzelle mit der flüssigen Phase entsprechend dem Druckprofil beaufschlagbar ist. Das Schaltelement kann ein fluidisches Schaltelement (insbesondere ein mit einer Steuereinrichtung steuerbares bzw. schaltbares Fluidventil) in einem fluidischen Pfad sein, das selektiv einen Fluidfluss und einen zumindest partiellen Druckausgleich durch den fluidischen Pfad zulässt oder unterbindet, je nach Schaltzustand.

Gemäß einem Ausführungsbeispiel der Erfindung kann das Schaltelement zwischen dem Druckbehälter und der Messzelle angeordnet sein. Dadurch kann der Druck auf den Druckbehälter angelegt werden, bis dieser einen gewünschten Anfangswert angenommen hat. Durch nachfolgendes Schalten des Schaltelements zum Bringen des Druckbehälters in Fluidkommunikation mit der Messzelle kann dann in definierter Weise das Druckprofil an die Messzelle angelegt werden.

Gemäß einem Ausführungsbeispiel der Erfindung kann während der Druckänderung alternierend zwischen einem eine Fluidverbindung zwischen dem Druckbehälter und der Messzelle zulassenden Betriebsmodus und einem eine Fluidverbindung zwischen dem Druckbehälter und der Messzelle unterbindenden Betriebsmodus gewechselt werden, um in dem die Fluidverbindung unterbindenden Betriebsmodus, insbesondere mehrere Male, ein Basisliniensignal zu erfassen und damit ein in dem die Fluidverbindung zulassenden Betriebsmodus erfasstes Messsignal zu korrigieren. Dies kann durch ein entsprechendes Schalten des Schaltelements zwischen dem Druckbehälter und der Messzelle erreicht werden.

Gemäß einem Ausführungsbeispiel der Erfindung kann die Druckerzeugungseinheit aus einer Gruppe ausgewählt sein, die besteht aus einer Pumpe, insbesondere einer Membranpumpe, und/oder einer Gasdruckversorgung, insbesondere einer Gasflasche (zum Beispiel eine Stickstoffflasche). Die Druckerzeugungseinheit kann über ein weiteres Fluidventil selektiv bzw. steuerbar mit dem Druckbehälter fluidisch koppelbar sein oder davon fluidisch entkoppelbar sein.

Gemäß einem Ausführungsbeispiel der Erfindung kann die Vorrichtung eine Druckmesseinheit zum Erfassen des Druckverlaufs entsprechend dem beaufschlagten Druckprofil aufweisen, insbesondere stromaufwärts von oder an dem Druckbehälter. Gemäß einem Ausführungsbeispiel der Erfindung kann das Schaltelement mittels einer Steuereinrichtung, welcher von der Druckmesseinheit der erfasste Druckwert übermittelt wird, derart schaltbar sein, dass das Schaltelement bei mittels der Druckmesseinheit festgestellter Überschreitung eines vorgebbaren Druckwerts die Fluidverbindung zwischen dem Druckbehälter und der Messzelle aktiviert. Der Druckwert bzw. Druckschwellwert kann der gewünschte Anfangswert des Druckprofils sein, mit dem die flüssige Phase zu Beginn der Zetapotenzialmessung durch die Messzelle gefördert wird.

Gemäß einem Ausführungsbeispiel der Erfindung kann der Vorratsbehälter überdruckfrei konfiguriert sein, insbesondere dessen Inneres auf Atmosphärendruck oder einem (gegenüber dem Druckbehälter) anderen Niederdruck befindlich ist. Somit kann der Vorratsbehälter druckentlastet bzw. auf ein Druckniveau gebracht sein, das durch die reservoirartigen Druckverhältnisse in der Umgebung definiert ist. Auf diese Weise kann, wenn sich an der Messzelle eingangsseitig der Druckwert kontinuierlich abbaut, ausgangsseitig ein Konstantdruck vorgegeben sein, wodurch im Ergebnis das sich zeitlich kontinuierlich abbauende Druckprofil einstellt.

Gemäß einem Ausführungsbeispiel der Erfindung kann die Erfasseinrichtung zum Erfassen der für das Zetapotenzial indikativen Information basierend auf Gleichung 1 und Gleichung 2 ausgebildet sein. Die in der Einleitung dieser Beschreibung dargestellte Auswertemethodik ist somit auch auf Ausführungsbeispiele der Erfindung anwendbar und soll insoweit auch als im Zusammenhang mit Ausführungsbeispielen der Erfindung offenbart verstanden werden.

Gemäß einem Ausführungsbeispiel der Erfindung kann die Erfasseinrichtung zum Erfassen der für das Zetapotenzial indikativen Information basierend auf einer Strommessung und basierend auf einer Spannungsmessung an der Messzelle eingerichtet sein. Basierend auf dem Wert des elektrischen Stroms und der elektrischen Spannung an einer Messzelle aus einer flüssigen Phase gemischt mit einer festen Phase kann das für die Grenzflächeneigenschaften indikative Zetapotenzial bestimmt werden.

Gemäß einem Ausführungsbeispiel der Erfindung kann die Vorrichtung eine Eingangselektrode an einem Eingang der Messzelle und eine Ausgangselektrode an einem Ausgang der Messzelle aufweisen, wobei die Erfasseinrichtung zum Erfassen der für das Zetapotenzial indikativen Information zwischen der Eingangselektrode und der Ausgangselektrode angeordnet ist. Eingangselektrode und/oder Ausgangselektrode kann/können als Elektrode erster Art oder Elektrode zweiter Art ausgebildet sein. Gemäß einem Ausführungsbeispiel der Erfindung kann die Eingangselektrode zum Wechselwirken zumindest mit der flüssigen Phase an dem Eingang der Messzelle und die Ausgangselektrode zum Wechselwirken zumindest mit der flüssigen Phase an dem Ausgang der Messzelle angeordnet sein.

Gemäß einem Ausführungsbeispiel der Erfindung kann die Erfasseinrichtung ausgebildet sein, mittels der Eingangselektrode und mittels der Ausgangselektrode (insbesondere zusätzlich) eine für einen elektrischen Widerstand der festen Phase und der flüssigen Phase in der Messzelle indikative Information zu erfassen. Gemäß einem Ausführungsbeispiel der Erfindung kann die Erfasseinrichtung ausgebildet sein, basierend auf der für den elektrischen Widerstand indikativen Information zumindest eine Information abzuleiten, die aus einer Gruppe ausgewählt ist, die besteht aus Information hinsichtlich des Vorliegens oder Nichtvorliegens von Gasblasen in der Messzelle, und einem Beitrag einer der Phasen (insbesondere der festen Phase) zur elektrischen Gesamtleitfähigkeit. Der elektrische Widerstand kann die Basis für weitergehende Informationen im Zusammenhang mit der Charakterisierung der Fest-Flüssig-Grenzfläche liefern.

Gemäß einem Ausführungsbeispiel der Erfindung kann die Vorrichtung einen Flüssigphasenrückfördermechanismus zum Rückfördern von flüssiger Phase von dem Vorratsbehälter in den Druckbehälter aufweisen. Auf diese Weise kann die vermessene flüssige Phase rückgeführt und wiederverwendet werden. Dadurch ist auch eine mehrmalige Vermessung einer flüssigen Probe ermöglicht, so dass geringere Mengen flüssiger Probe ausreichend sind, und sind die anfallenden Abfallmengen gering gehalten. Der Flüssigphasenrückfördermechanismus kann insbesondere eine Pumpe, die zum Druckbeaufschlagen des Druckbehälters eingesetzt wird, synergistisch zum Rückfördern mitverwenden (insbesondere in einem Betrieb mit umgekehrter Förderrichtung).

Gemäß einem Ausführungsbeispiel der Erfindung kann das Schaltelement in einem fluidischen Pfad angeordnet sein, in dem mittels des Flüssigphasenrückfördermechanismus flüssige Phase von dem Vorratsbehälter zu dem Druckbehälter rückförderbar ist. Somit kann auch das Schaltelement synergistisch mitverwendet werden, um nicht nur durch einen Schaltvorgang den Beginn der Messung der für das Zetapotenzial indikativen Information vorzugeben, sondern in einem anderen Schaltzustand ohne das Erfordernis weiterer Schaltelemente oder zusätzlicher Fluidleitungen auch ein Rückfördern vermessender flüssiger Phase in den Druckbehälter zu ermöglichen.

Gemäß einem Ausführungsbeispiel der Erfindung kann die Vorrichtung ferner einen weiteren Druckbehälter, in dem weitere flüssige Phase aufnehmbar ist, und eine weitere Messzelle aufweisen, die stromabwärts von und in Fluidkommunikation mit dem weiteren Druckbehälter bringbar angeordnet ist und in der weitere feste Phase aufnehmbar ist, wobei die Druckbeschlageinrichtung zum Beaufschlagen des weiteren Druckbehälters mit einem Druckprofil mit einer zeitlich kontinuierlichen Druckänderung derart eingerichtet ist, dass dadurch weitere flüssige Phase aus dem weiteren Druckbehälter durch die weitere Messzelle förderbar ist, und wobei die Erfasseinrichtung zum Erfassen von für das Zetapotenzial indikativer Information an der weiteren Messzelle während des Beaufschlagens des weiteren Druckbehälters mit dem Druckprofil ausgebildet ist. Auf diese Weise kann ein und dieselbe Druckerzeugungseinrichtung und gegebenenfalls ein und derselbe Vorratsbehälter für mehrere Messpfade verwendet werden, womit eine besonders kompakte Vorrichtung erhalten wird.

Gemäß einem Ausführungsbeispiel der Erfindung kann die Erfasseinrichtung ausgebildet sein, einen fluidischen Pfad aus dem weiterem Druckbehälter und der weiteren Messzelle als Referenzmesspfad für einen Messpfad aus dem Druckbehälter und der Messzelle einzusetzen. Durch einen solchen Referenz- oder einen Kalibrierungspfad kann die Genauigkeit und Zuverlässigkeit der eigentlichen Hauptmessung verbessert werden.

Gemäß einem Ausführungsbeispiel der Erfindung kann ein fluidischer Pfad aus dem weiteren Druckbehälter und der weiteren Messzelle als zusätzlicher Messpfad zum Ermitteln von für ein Zetapotenzial indikativer Information zum Charakterisieren einer Grenzfläche zwischen der weiteren festen Phase und der weiteren flüssigen Phase ausgebildet sein. Mit einer kompakten Vorrichtung, die sich zumindest ein Teil ihrer Komponenten für mehrere Messaufgaben teilen kann, kann auf engem Raum und mit gut vergleichbaren Bedingungen eine Messung mehrerer Proben parallel oder sequenziell durchgeführt werden.

Gemäß einem Ausführungsbeispiel der Erfindung kann die Erfasseinrichtung zum Erfassen der für das Zetapotenzial indikativen Information unter Durchführung einer Basislinienkorrektur, insbesondere einer Korrektur einer sich zeitlich ändernden Basislinie, eingerichtet sein. Vorzugsweise können Spannungswerte bei kontinuierlich verändertem Druckprofil (d.h. das Strömungspotenzial bei anliegendem Druck) und der Basislinie (Asymmetriepotenzial bei Differenzdruck Null) durch eine Modellfunktion (zum Beispiel durch eine Polynomfunktion, insbesondere zweiten Grades) modelliert werden. Der zeitliche Drift der Basislinie kann dann vom zeitlichen Verlauf des Strömungspotenzials bei abnehmender Druckdifferenz subtrahiert werden. Mit solch einer Basislinienkorrektur kann die Genauigkeit und Verlässlichkeit der ermittelten Messergebnisse weiter verbessert werden.

Gemäß einem Ausführungsbeispiel der Erfindung kann die Basislinienkorrektur unter Berücksichtigung eines Asymmetriepotenzials einer Eingangselektrode an einem Eingang der Messzelle und einer Ausgangselektrode an einem Ausgang der Messzelle erfolgen. Insbesondere kann die Basislinienkorrektur unter Berücksichtigung einer zeitlichen Änderung des Asymmetriepotenzials hinsichtlich eines Zusammenhangs zwischen einem Strömungspotenzial oder einem Strömungsstrom einerseits und einem Differenzdruck andererseits erfolgen. Gemäß einem Ausführungsbeispiel der Erfindung kann die Erfasseinrichtung zum Erfassen eines von der festen Phase und der flüssigen Phase unabhängigen, insbesondere auf einem Asymmetriepotenzial einer Eingangselektrode an einem Eingang der Messzelle und einer Ausgangselektrode an einem Ausgang der Messzelle beruhenden, Basissignals während des Beaufschlagens des Druckbehälters mit dem Druckprofil eingerichtet sein.

Die Berücksichtigung des Asymmetriepotenzials der Messelektroden (auch als Basislinienkorrektur bezeichnet) und dessen zeitlicher Änderung in der Auswertung der Druckrampen (Zusammenhang zwischen Strömungspotenzial oder Strömungsstrom und Differenzdruck) ist vorteilhaft für eine hochpräzise und eindeutige Interpretation insbesondere in den folgenden Szenarien:

- gemessene Druckrampen bei hoher Ionenstärke
- gemessene Druckrampen in unmittelbarer Nähe des isoelektrischen Punktes (unabhängig von der gewählten Ionenstärke)
- gemessene Druckrampen an metallischen Materialoberflächen, sowie
- Änderung von Strömungspotenzial oder Strömungsstrom während eines Adsorptions- oder Desorptionsvorgangs

Insbesondere in den genannten kritischen Szenarien führt die Berücksichtigung einer Basislinienkorrektur durch die beschriebene Vorrichtung zu einer besonders genauen Bestimmung des Zetapotenzials (d.h. mit einem sehr kleinen Messfehler). In anderen Szenarien hingegen kann die Basislinienkorrektur auch weggelassen werden.

Insbesondere kann in den angeführten Beispielen der Wert des tatsächlichen Messsignals von Strömungspotenzial oder -strom mitunter kleiner als jener des Asymmetriepotenzials (Basislinie) sein. Bei einer zeitlich konstanten Basislinie ist eine Korrektur nicht notwendig, um trotz dieser ungünstigen Bedingungen (Messsignal kleiner Basissignal) ein plausibles Ergebnis (Steigung der Druckrampe) zu erhalten. Allerdings können die erwähnten Einflussgrößen (insbesondere Polarisierbarkeit der Elektroden, Strömung an den Elektrodenoberflächen, thermischer Drift der Elektronik, Einfluss von Adsorbat) unter ungünstigen Umständen zu einer nicht reproduzierbaren zeitlichen Änderung dieses Basissignals führen. Neben der Änderung des Strömungspotenzials oder -stroms während des Druckgefälles ist die Kenntnis (zum Beispiel basierend auf entsprechenden Messungen) des Basissignals während der Messzeit dann sehr vorteilhaft.

Vorteile einer Vorrichtung bzw. eines Verfahrens gemäß exemplarischen Ausführungsbeispielen der Erfindung liegen erstens darin, dass ein komprimiertes Gasvolumen selbständig expandiert und somit während der Messung keine Druckimpulse (und in der Folge keine Messartefakte, zum Beispiel durch eine Pumpe oder durch Regelung eines ständig angelegten externen Gasdrucks) erzeugt werden. Zweitens ermöglicht unter ungünstigen Rahmenbedingungen, mithin optional, gerade die Kombination einer solchen Vorrichtung bzw. eines solchen Verfahrens mit einer parallelen Messung von Strömungspotenzial- oder -strom und der zeitlichen Änderung des Basissignals auch die Anwendbarkeit der Methode unter Messbedingungen, die zu extrem kleinen Messwerten führen (siehe die oben genannten Anwendungsbereiche).

Besonders vorteilhaft sind exemplarische Ausführungsbeispiele, insbesondere mit der Basislinienkorrektur, daher in folgenden Anwendungsfällen anwendbar:

- Ermitteln der für das Zetapotenzial indikativen Information bei hoher Ionenstärke, insbesondere bei einer Ionenstärke von mindestens ungefähr 0.001 mol/l, weiter insbesondere bei einer Ionenstärke von mindestens ungefähr 0. 1 mol/l
- -Ermitteln der für das Zetapotenzial indikativen Information während eines Adsorptionsprozesses oder während eines Desorptionsprozesses (insbesondere an der festen Phase)
- -Ermitteln der für das Zetapotenzial indikativen Information an einer metallischen Materialoberfläche (insbesondere der festen Phase)
- -Ermitteln der für das Zetapotenzial indikativen Information in unmittelbarer Nähe des isoelektrischen Punktes (d.h. des pH-Werts der flüssigen Phase, bei dem sich positive Ladungen und negative Ladungen ausgleichen), insbesondere bei höchstens ungefähr fünf Prozent Abweichung des pH-Werts von dem isoelektrischen Punkt.

Gemäß einem Ausführungsbeispiel kann die Druckbeaufschlagungseinrichtung zum Beaufschlagen des Druckbehälters mit einem Druckprofil derart eingerichtet sein, dass eine Druckdifferenz zwischen einem Anfangsdruckwert an dem Druckbehälter und einem Anfangsdruckwert an dem Vorratsbehälter größer als ungefähr eine Atmosphäre ist, insbesondere größer als ungefähr 2 bar ist, weiter insbesondere zwischen ungefähr 5 bar und ungefähr 10 bar ist. Anschaulich legt die Druckbeaufschlagungseinrichtung an den Druckbehälter vorzugsweise einen Überdruck an (anstatt abzusaugen). Dadurch können auch sehr hohe Differenzdrücke erhalten werden und kann somit der Bereich durchführbarer Messungen (insbesondere hinsichtlich vermessbarer Materialien) erweitert werden.

Im Folgenden werden exemplarische Ausführungsbeispiele der vorliegenden Erfindung mit Verweis auf die folgenden Figuren detailliert beschrieben.

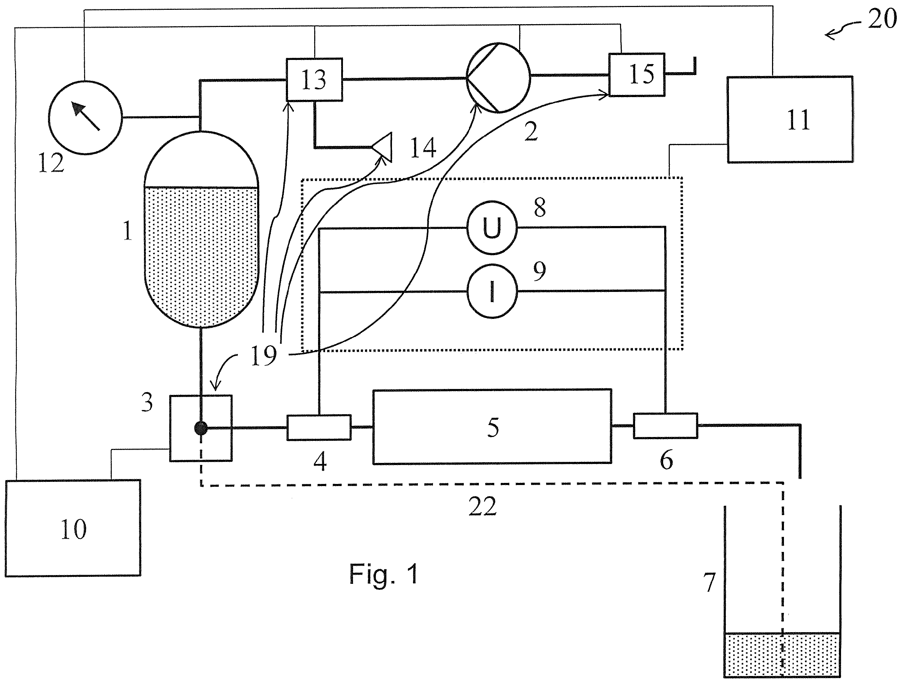

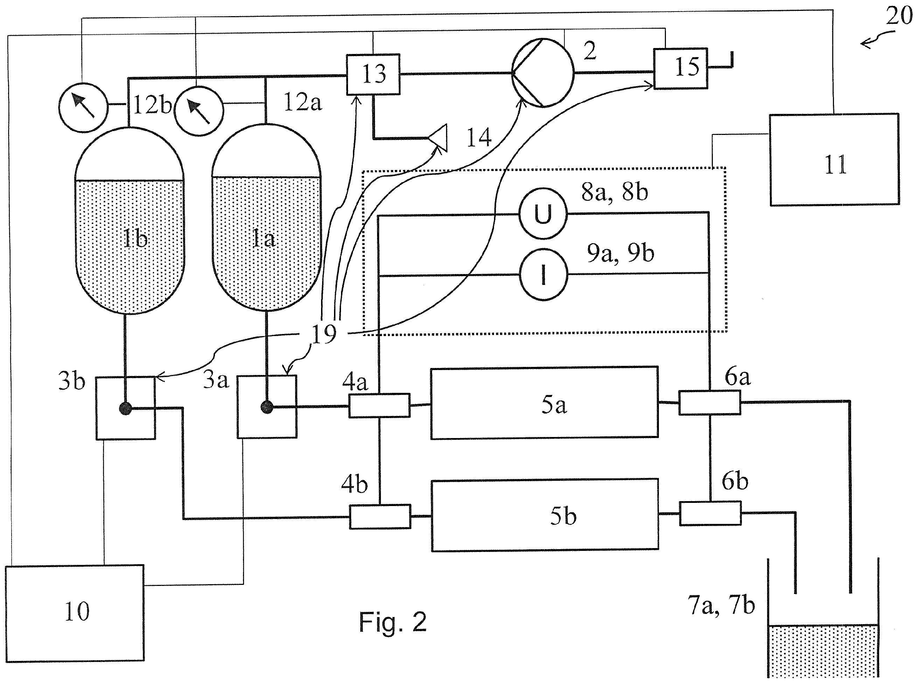

Figur 1 und Figur 2 zeigen Vorrichtungen gemäß exemplarischen Ausführungsbeispielen der Erfindung zum Ermitteln eines Zetapotenzials einer Probe mit einer festen Phase und mit einer flüssigen Phase, um Informationen über diese Probe zu erhalten, insbesondere Informationen über eine Grenzfläche bzw. eine Wechselwirkung zwischen der festen Phase und der flüssigen Phase.

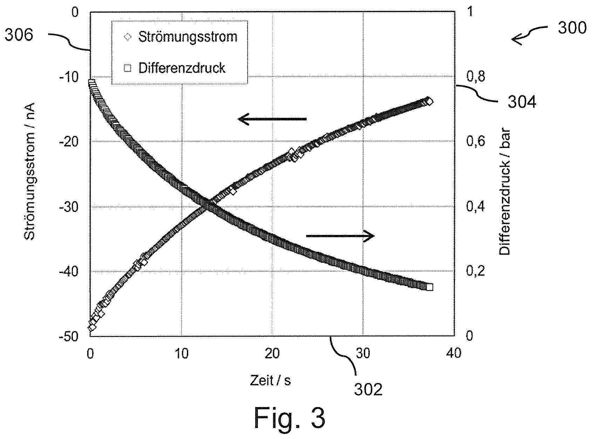

Figur 3 zeigt ein Diagramm, das als Beispiel eine Messung von Strömungsstrom im Druckbereich 150-800 mbar zeigt und eine Abhängigkeit zwischen der Zeit, einer Druckdifferenz und dem Strömungsstrom gemäß exemplarischen Ausführungsbeispielen der Erfindung zeigt.

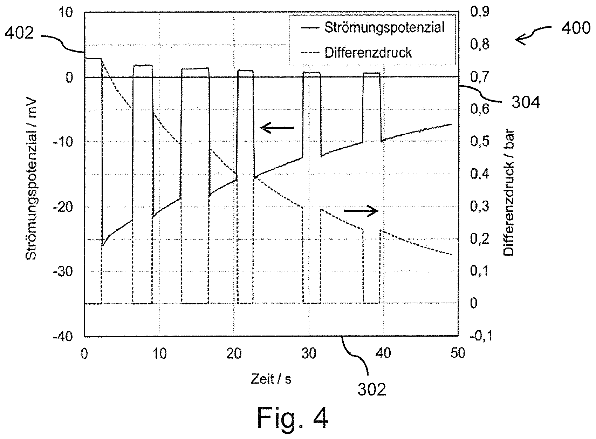

Figur 4 zeigt ein Diagramm, das als Beispiel die Messung des Strömungspotenzials während der kontinuierlichen Abnahme des Differenzdrucks und die in Serie geschaltete Messung der zeitlichen Änderung des Asymmetriepotenzials zeigt und eine Abhängigkeit zwischen der Zeit, einer Druckdifferenz und dem Strömungspotenzial gemäß exemplarischen Ausführungsbeispielen der Erfindung zeigt.

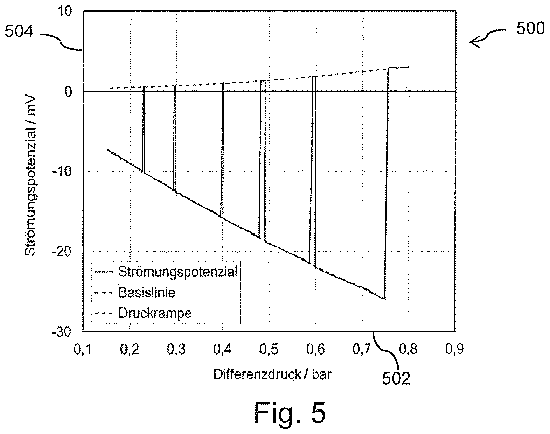

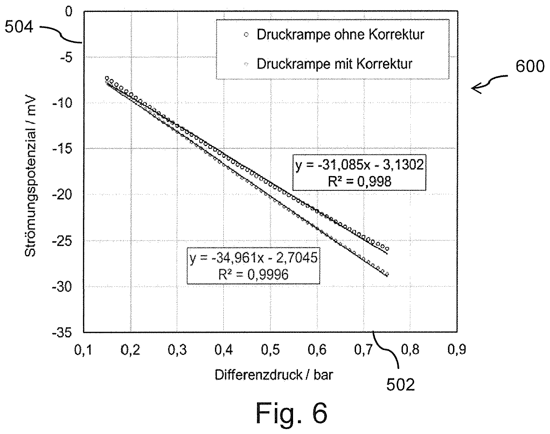

Figur 5 und Figur 6 zeigen Diagramme, die ein Verfahren zur Korrektur einer Basislinie gemäß exemplarischen Ausführungsbeispielen der Erfindung dadurch verdeutlichen, dass das Strömungspotenzial (bei anliegendem Differenzdruck) bzw. das Asymmetriepotenzial (bei $\Delta p$ = 0 bar) gegen den Differenzdruck aufgetragen ist.

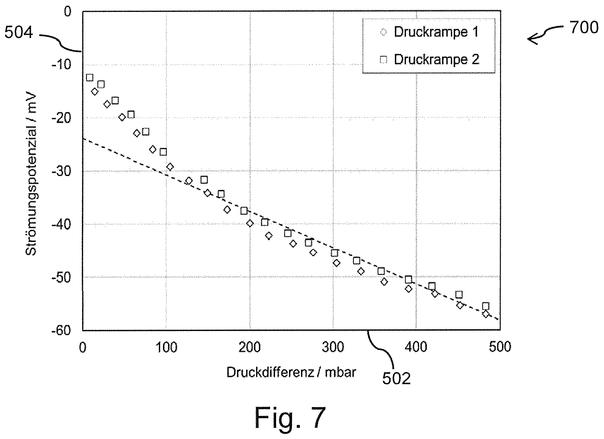

Figur 7 zeigt ein Diagramm, das für das Beispiel einer Druckrampenmessung für eine leitfähige Probe in Gegenwart einer 0.001 mol/l KCl Lösung eine Abhängigkeit zwischen einer Druckdifferenz und dem Strömungspotenzial zeigt.

Gleiche oder ähnliche Komponenten in unterschiedlichen Figuren sind mit gleichen Bezugsziffern versehen.

Bevor bezugnehmend auf die Figuren exemplarische Ausführungsbeispiele der Erfindung beschrieben werden, sollen noch einige allgemeine Aspekte der Erfindung erläutert werden:

Bei einer Druckrampenmessung wird das Strömungspotenzial $U_{str}(\Delta p)$ simultan zu einer kontinuierlich ansteigenden Druckdifferenz gemessen und der Strömungspotenzialkoeffizient $dU_{str}/d\Delta p$ in Gleichung 2 als Steigung der linearen Regression der Messpunkte ermittelt. Die Druckrampenmessung hat gegenüber einer Druckstufenmessung den Vorteil der größeren Anzahl an individuellen Messpunkten (Erhöhung der Qualität des Messergebnisses) und der wesentlich kürzeren Messzeit.

Die Messung des Strömungspotenzials und des Strömungsstroms nach herkömmlichen Messmethoden beschränkt sich auf die Bestimmung des Zetapotenzials bei geringen Ionenstärken ($I$ < 0.1 mol/l). Mit zunehmender Ionenstärke verringert sich das Messsignal insbesondere des Strömungspotenzials (annähernd doppelt-exponentielle Abnahme) und auch des Strömungsstroms (annähernd exponentielle Abnahme). Damit nähert sich das bei einer bestimmten Druckdifferenz entstehende Strömungspotenzial der Größenordnung des Asymmetriepotenzials. Neben den Beiträgen zur Elektrodenpolarisation treten in diesem Größenbereich weitere Effekte auf, die für die Messung kleiner Signale zu berücksichtigen sind:

Ein solcher Effekt ist die zeitliche Abhängigkeit der Elektrodenpolarisation. Für Messungen des Strömungspotenzials und des Strömungsstroms insbesondere in Gegenwart hoher Ionenstärken ($I \geq 0.1$ mol/l) ist es nicht mehr ohne Weiteres zulässig, ein zeitlich stabiles Asymmetriepotenzial aufgrund von Elektrodenpolarisation anzunehmen. Vielmehr ist häufig zu beobachten, dass sich zeitlich ändernde Polarisationseffekte auch auf die Stabilität des Messsignals auswirken. Der Einfluss elektrisch leitfähiger Materialien, beispielsweise Metalle, auf das Asymmetriepotenzial und seine zeitliche Instabilität sollte ebenfalls Rechnung getragen werden.

Figur 7 zeigt das Beispiel einer Druckrampenmessung für eine leitfähige Probe in Gegenwart einer 0.001 mol/l KCl Lösung. Trotz der geringen Ionenstärke ($I$ = 0.001 mol/l) ist hier eine signifikante Abweichung vom erwarteten linearen Zusammenhang zwischen Strömungspotenzial und Differenzdruck zu erkennen. Die Ursache liegt in einer zeitlichen Änderung des Asymmetriepotenzials (Basislinie) während der Druckrampenmessung.

Auch die strömungsabhängige Elektrodenpolarisation ist zu berücksichtigen. Bei hoher Ionenstärke und daher bei geringen Messwerten des Strömungspotenzials und Strömungsstroms werden zusätzlich zu Beiträgen zur Elektrodenpolarisation Polarisationseffekte beobachtet, die strömungsabhängig sind (siehe Vinogradov J, Jaafar M Z, Jackson M D (2010) "Measurement of streaming potenzial coupling coefficient in sandstones saturated with natural and artificial brines at high salinity", J Geophys Res 115: B1 2204). Eine Lösung zur Vermeidung dieses Effekts ist die Verwendung von externen Elektroden, die über eine Salzbrücke mit der für die Messung von Strömungspotenzial und Strömungsstrom verwendeten Elektrolytlösung verbunden sind. Der Nachteil dieser Methode liegt in der verringerten Empfindlichkeit und zeitlichen Ansprechzeit der externen Messelektroden.

Auch Effekte der Elektronik tragen bei. Die Werte des Strömungspotenzials und des Strömungsstroms nähern sich in Gegenwart von Elektrolytlösungen mit hoher Ionenstärke der Auflösungsgrenze der entsprechenden Messbereiche. Das Signal/Rauschverhältnis wird daher auch von elektronischer Drift, beispielsweise aufgrund von Temperaturschwankungen, mitbestimmt.

Gemäß exemplarischen Ausführungsbeispielen kann eine Vorrichtung und ein Verfahren zur Bestimmung des Zetapotenzials an Festkörperoberflächen mit einer Korrektur der Basislinie geschaffen werden. Eine Korrektur der beschriebenen Einflüsse ist für die korrekte Messung des Strömungspotenzials und Strömungsstroms insbesondere bei hoher Ionenstärke vorteilhaft.

Zum einen werden gemäß einem exemplarischen Ausführungsbeispiel Pulsationseffekte berücksichtigt bzw. unterdrückt. Die Verwendung einer mechanischen Pumpe für das Aufbringen der Druckdifferenz zur Messung ein oder mehrerer Druckstufen und Druckrampen führt zu Pulsationen in der Flüssigkeitsströmung und damit in der Druckdifferenz, die sich unmittelbar auf das Signal des Strömungspotenzials und des Strömungsstroms auswirken. Für die Bestimmung des Zetapotenzials aus Messungen des Strömungspotenzials und Strömungsstroms ist daher für den gesamten Anwendungsbereich der Ionenstärke, insbesondere jedoch bei hoher Ionenstärke, eine pulsationsfreie Aufbringung der Druckdifferenz sehr vorteilhaft. Zur Kompensation der zeitabhängigen Einflüsse von Elektrodenpolarisation und anderen Drifterscheinungen auf Spannungs- und Stromwerte im stationären Zustand (Basislinie) wird eine pulsationsfreie und zeitlich kontinuierliche Änderung der Druckdifferenz ermöglicht.

**Figur 1** zeigt eine Vorrichtung 20 gemäß einem exemplarischen Ausführungsbeispiel der Erfindung zum Ermitteln von für ein Zetapotenzial indikativer Information, um eine Grenzfläche zwischen einer festen Phase (als zu untersuchende Probe) und einer flüssigen Phase (insbesondere eine Testflüssigkeit) zu charakterisieren.

Die Vorrichtung 20 weist einen Druckbehälter 1 auf, in dem die flüssige Phase (das heißt eine zu untersuchende Flüssigkeit) aufnehmbar ist. Der Druckbehälter 1 ist stromaufwärts davon fluidisch an ein Ventil 13 und an eine Druckmesseinheit 12 angeschlossen und ist stromabwärts an ein Schaltelement 3 (konfiguriert als ein weiteres fluidisches Ventil) angeschlossen. Ansonsten ist der Druckbehälter 1 gegenüber der Umgebung hermetisch abgedichtet bzw. druckentkoppelt, so dass im Inneren des Druckbehälters 1 ein von dem umgebenden Atmosphärendruck unterschiedlicher Druck herrschen bzw. eingestellt werden kann.

Ferner weist die Vorrichtung 20 eine Messzelle 5 auf, die bezogen auf eine bestimmungsgemäße Flussrichtung der Flüssigkeit stromabwärts von dem Druckbehälter 1 angeordnet ist und in Fluidkommunikation mit dem Druckbehälter 1 gebracht werden kann, wenn das Schaltelement 3 von einer Steuereinrichtung 10 in einem diese Fluidkommunikation zulassenden Betriebszustand geschaltet ist, der auch als Durchlasszustand bezeichnet werden kann. Schaltet die Steuereinrichtung 10 das Schaltelement 3 dagegen in einen Sperrzustand, ist eine Fluidkommunikation zwischen dem Druckbehälter 1 und der Messzelle 5 vorübergehend verunmöglicht. In der Messzelle 5 ist die feste Phase (d.h. der zu untersuchende Festkörper, dessen Wechselwirkung insbesondere mit der zu untersuchenden Flüssigkeit analysiert werden soll) aufnehmbar bzw. aufgenommen.

Ein offener, überdruckfreier Vorratsbehälter 7, der auf Atmosphären- bzw. Umgebungsdruck befindlich ist, ist stromabwärts von der Messzelle 5 angeordnet. Da sich der Vorratsbehälter 7 in Fluidkommunikation mit der Messzelle 5 befindet, kann die flüssige Phase nach Durchströmen der Messzelle 5 aus dieser ausgelassen und in dem Vorratsbehälter 7 aufgefangen bzw. aufgenommen werden. Der Vorratsbehälter 7 ist ein offener Behälter und somit mit der umgebenden Atmosphäre verbunden.

Die Vorrichtung 20 weist ferner eine Druckbeaufschlagungseinrichtung 19 auf, die durch eine Mehrzahl miteinander zusammenwirkender Komponenten gebildet ist. Die Druckbeaufschlagungseinrichtung 19 ist zum Beaufschlagen des Druckbehälters 1 mit einem vorgebbaren Druckprofil mit einer zeitlich kontinuierlichen Druckänderung eingerichtet. Dies bedeutet, dass dem Druckbehälter 1 und in der Folge der Messzelle 5 ein mittels der Steuereinrichtung 10 (zum Beispiel ein Prozessor oder ein Teil eines Prozessors) gesteuerter, zeitlich veränderlicher Druckwert aufgeprägt wird, der ausgehend von einem Anfangswert während der Messung zum Erfassen der für das Zetapotenzial indikativen Information zum Beispiel kontinuierlich bzw. stetig abnimmt (zum Beispiel über der Zeit linear abnimmt). Unter dem Einfluss dieses zeitlich veränderlichen und kontinuierlich abnehmbaren Druckprofils wird kontinuierlich flüssige Phase aus dem Druckbehälter 1 durch die Messzelle 5 in den Vorratsbehälter 7 gefördert. Anschaulich nimmt der Druck in dem Druckbehälter 1 mit der Zeit kontinuierlich ab, während der Druck in dem Vorratsbehälter 7 konstant auf Atmosphärendruck verbleibt. Indem die Druckbeaufschlagungseinrichtung 19 der Messzelle 5 ein Druckprofil mit einer pulsationsfreien Druckänderung bereitstellt, sind Messartefakte unterdrückt oder eliminiert, so dass auch unmittelbare und ungewollte Änderungen im Strömungspotenzial oder Strömungsstrom im Flüssigkeitsstrom unterdrückt oder eliminiert sind. Dies erhöht die Messgenauigkeit und die Reproduzierbarkeit der Messung, da Störeinflüsse ausgeblendet oder zumindest stark reduziert werden können. Die Druckbeaufschlagungseinrichtung 19 ist eingerichtet, einen Gasraum oberhalb der flüssigen Phase in dem Druckbehälter 1 mit einem komprimierten Gaspolster zu beaufschlagen, das auf die Flüssigkeitsoberfläche drückt, um dadurch das Druckprofil mit einer kontinuierlichen Abnahme des Drucks zu erzeugen. Zu diesem Zweck weist die Druckbeaufschlagungseinrichtung 19 eine Druckerzeugungseinheit (im gezeigten Ausführungsbeispiel ausgeführt durch eine Pumpe 2 (zum Beispiel eine Membranpumpe) stromabwärts eines weiteren fluidischen Ventils 15 bzw. eine Gasdruckversorgung 14 (zum Beispiel eine Stickstoffflasche)) zum Erzeugen von Druck auf. Auch das Schaltelement 3 bildet einen Teil der Druckbeaufschlagungseinrichtung 19 und ist mittels der Steuereinrichtung 10 derart schaltbar, dass mittels Schaltens des Schaltelements 3 zum fluidischen Koppeln der Druckerzeugungseinheit 2 bzw. 14 mit der Messzelle 5 die Messzelle 5 mit der flüssigen Phase entsprechend dem gesteuert vorgegebenen Druckprofil beaufschlagt wird.

Die Vorrichtung 20 weist ferner die Druckmesseinheit 12 zum Erfassen des Druckverlaufs entsprechend dem beaufschlagten Druckprofil direkt stromaufwärts von dem Druckbehälter 1 auf. Ein erfasster Druckwert wird von der Druckmesseinheit 12 der Steuereinrichtung 10 und einer Erfasseinrichtung 11 übermittelt. Gesteuert durch die Steuereinrichtung 10 wird das Schaltelement 3 dann derart geschaltet, dass das Schaltelement 3 bei mittels der Druckmesseinheit 12 festgestellter Überschreitung eines vorgebbaren Druckwerts (der dem Anfangswert des aufzuprägenden Druckprofils entsprechen kann) die Fluidverbindung zwischen dem Druckbehälter 1 und der Messzelle 5 aktiviert. Nach diesem Schalten des Schaltelements 3 wird dieser erzeugte Druck verwendet, um die flüssige Phase aus dem Druckbehälter 1 durch die Messzelle 5 in den durchgehend auf Atmosphärendruck befindlichen Vorratsbehälter 7 zu überführen. Dadurch reduziert sich kontinuierlich und pulsationsfrei der Differenzdruck zwischen Druckbehälter 1 und Vorratsbehälter 7 bzw. zwischen einem Eingang und einem Ausgang der Messzelle 5.

Eine Erfasseinrichtung 11 (zum Beispiel ein Prozessor oder ein Teil eines Prozessors) ist zum Erfassen der für das Zetapotenzial indikativen Information an der Messzelle 5 während des Beaufschlagens des Druckbehälters 1 mit dem Druckprofil ausgebildet. Die Erfasseinrichtung 11 ist zum Erfassen der für das Zetapotenzial indikativen Information basierend auf einer Strommessung (siehe Bezugszeichen 9 in Figur 1) und basierend auf einer Spannungsmessung (siehe Bezugszeichen 8 in Figur 1) an der Messzelle 5 eingerichtet. Die Vorrichtung 20 weist hierzu eine Eingangselektrode 4 an einem Eingang der Messzelle 5 und eine Ausgangselektrode 6 an einem Ausgang der Messzelle 5 auf, wobei die Erfasseinrichtung 11 zum Erfassen von Signalen bzw. Messwerten zwischen der Eingangselektrode 4 und der Ausgangselektrode 6 angeordnet ist. Die Eingangselektrode 4 ist zum Wechselwirken mit der flüssigen Phase an dem Eingang der Messzelle 5 und die Ausgangselektrode 6 ist zum Wechselwirken mit der flüssigen Phase an dem Ausgang der Messzelle 5 angeordnet. Die Erfasseinrichtung 11 erfasst mittels der Eingangselektrode 4 und mittels der Ausgangselektrode 6 optional auch eine für einen elektrischen Widerstand der festen Phase und der flüssigen Phase in der Messzelle 5 indikative Information.

Basierend auf dieser Information kann ermittelt werden, ob sich in der Messzelle 5 unerwünschte Gasblasen befinden, welche die Messung des Zetapotenzials verfälschen würden. Auch kann diese Information herangezogen werden, um einem Beitrag der festen Phase zur elektrischen Gesamtleitfähigkeit zu ermitteln.

Die Vorrichtung 20 weist ferner einen Flüssigphasenrückfördermechanismus zum Rückfördern von flüssiger Phase von dem Vorratsbehälter 7 in den Druckbehälter 1 auf. Der Flüssigphasenrückfördermechanismus ist durch eine Fluidleitung 22 gebildet, deren eines Ende in die in dem Vorratsbehälter 7 aufgenommene, bereits vermessene flüssige Phase eintaucht und deren anderes Ende durch das Schaltelement 3 in Fluidverbindung mit dem Druckbehälter 1 bringbar ist. Das Schaltelement 3 ist also in dem durch die Fluidleitung 22 konstituierten fluidischen Pfad angeordnet, in dem mittels des Flüssigphasenrückfördermechanismus flüssige Phase von dem Vorratsbehälter 7 zu dem Druckbehälter 1 rückförderbar ist. Die Pumpe 2 kann die Förderleistung zum Rückfördern der Flüssigkeit aufbringen und hierfür in umgekehrter Richtung fördern als zuvor.

Die in Figur 1 gezeigte Vorrichtung 20 ist zur pulsationsfreien Änderung der Druckdifferenz mit gleichzeitiger Messung des Strömungspotenzials und Strömungsstroms ausgebildet. Im Druckbehälter 1 wird der Druck im Gasraum oberhalb der Flüssigkeit mit der Pumpe 2, beispielsweise einer Membranpumpe, oder der Gasdruckversorgung 14, beispielsweise eine Stickstoffflasche, erhöht. Der erzeugte Druck wird über die Druckmesseinheit 12 gemessen. Anschließend öffnet das als Ventil ausgebildete Schaltelement 3, und die Flüssigkeit strömt durch die oder vorbei an der Eingangselektrode 4 in die Messzelle 5. Das Öffnen und Schließen des Schaltelements 3 und die Steuerung bzw. Regelung der Pumpe 2 erfolgt über die Steuereinrichtung 10. Die Festkörperprobe in der Messzelle 5 ist an entsprechenden Probenträgern derart befestigt, dass sich aus dem Probenmaterial ein Strömungskanal in Form einer Kapillare, beispielsweise mit einer rechteckigen Querschnittsfläche, bildet. Nach der Messzelle 5 strömt die Flüssigkeit durch die oder vorbei an der Ausgangselektrode 6 und wird in dem Vorratsbehälter 7 aufgefangen. Während dieses Vorgangs nimmt der Differenzdruck (d.h. Gasdruck in Druckbehälter 1 versus atmosphärischer Druck) sukzessive ab und ein Strömungspotenzial oder ein Strömungsstrom werden während des Durchtritts der Flüssigkeit durch den Strömungskanal in der Messzelle 5 erzeugt. Das Strömungspotenzial wird als Spannungswert zwischen der Eingangselektrode 4 und der Ausgangselektrode 6 über einen elektrischen Schaltkreis (siehe Bezugszeichen 8) gemessen. Der Strömungsstrom wird als Stromwert zwischen der Eingangselektrode 4 und der Ausgangselektrode 6 über einen weiteren elektrischen Schaltkreis (siehe Bezugszeichen 9) gemessen. Die gemessenen Werte werden an die Auswerteeinheit bzw. Erfasseinrichtung 11 zur Bestimmung des Zetapotenzials übermittelt.

Die in Figur 1 beschriebene Vorrichtung 20 kann auch dazu verwendet werden, Flüssigkeit aus dem Vorratsbehälter 7 mit Hilfe der Pumpe 2, beispielsweise einer Membranpumpe, durch eine entsprechende Schaltung des Schaltelements 3 in den Druckbehälter 1 zu überführen.

Figur 1 zeigt zusätzlich eine Ventilanordnung, die aus den Ventilen 13, 15 gebildet ist, zur Beaufschlagung von Druck im Druckbehälter 1 durch die Pumpe 2 oder die externe Gasdruckversorgung 14 und dem Erzeugen von Unterdruck für die Überführung bzw. Rückführung von Flüssigkeit aus dem Vorratsbehälter 7 in den Druckbehälter 1 (d.h. durch Ansaugen). Die Steuerung oder Regelung der Ventile 13, 15, die beispielsweise als mehrere 2-Wege-Ventile oder 3-Wege-Ventile ausgebildet sein können, erfolgt ebenfalls durch die Steuereinrichtung 10.

Die Eingangselektrode 4 und die Ausgangselektrode 6 werden auch verwendet, um den elektrischen Widerstand im Strömungskanal der Messzelle 5 zu messen. Der Widerstand dient vorwiegend der Kontrolle der luftblasenfreien Befüllung der Messzelle 5 und insbesondere des Strömungskanals, wird aber auch verwendet, um den Beitrag der Festkörperprobe zur elektrischen Gesamtleitfähigkeit im Strömungskanal zu bestimmen. Die Kenntnis der tatsächlichen Gesamtleitfähigkeit im Strömungskanal ermöglicht die Korrektur des nach der obigen Gleichung 2 berechneten Zetapotenzials im Fall von ionisch oder elektronisch leitfähigen Festkörperproben.

Die Druckbeaufschlagung der Messlösung mit einem komprimierten Gaspolster und die kontinuierliche Abnahme des Drucks nach dem Schalten des Schaltelements 3 ermöglicht eine pulsationsfreie Änderung der Druckdifferenz. Das pulsationsfreie Aufbringen der Druckdifferenz ist insbesondere bei einer sich zeitlich ändernden Druckdifferenz vorteilhaft, da Pulsationen im Flüssigkeitsstrom zu unmittelbaren und ungewollten Änderungen im Strömungspotenzial oder Strömungsstrom führen. Diese Schwankungen können zu einem systematischen Fehler in der Berechnung des Zetapotenzials führen.

Eine Basislinienkorrektur kann bei der Vorrichtung 20 gemäß Figur 1 die Messgenauigkeit in bestimmten Szenarien signifikant verbessern. Hierfür kann das Ventil 3 während des Druckabbaus entsprechend dem vorgegebenen Druckprofil alternierend zwischen seiner eine Fluidverbindung zulassenden Ventilstellung und seiner eine Fluidverbindung unterbindenden Ventilstellung geschaltet werden, um in der die Fluidverbindung unterbindenden Ventilstellung ein Basisliniensignal (insbesondere mehrere Male) zu erfassen und damit die Messung in der die Fluidverbindung zulassenden Ventilstellung zu korrigieren.

**Figur 2** zeigt eine Vorrichtung 20 zum Ermitteln von für ein Zetapotenzial an einer Grenzfläche zwischen einer festen Phase und einer flüssigen Phase indikativer Information gemäß einem anderen exemplarischen Ausführungsbeispiel der Erfindung. Die in Figur 2 gezeigte Vorrichtung 20 weist einige der in Figur 1 gezeigten Komponenten doppelt auf, wobei zur besseren Unterscheidung die in Figur 1 verwendeten Bezugszeichen in Figur 2 mit dem zusätzlichen Buchstaben "a" gekennzeichnet sind (zum Beispiel Druckbehälter 1a, der Druckbehälter 1 gemäß Figur 1 entspricht, etc.).

Die Vorrichtung 20 weist ferner einen weiteren Druckbehälter 1b auf, in dem weitere flüssige Phase aufnehmbar ist. Ferner enthält die Vorrichtung 20 gemäß Figur 2 zusätzlich eine weitere Messzelle 5b, die stromabwärts von und in Fluidkommunikation mit dem weiteren Druckbehälter 1b angeordnet ist und in der weitere feste Phase aufnehmbar ist. Die Druckbeschlageinrichtung 19 ist zum Beaufschlagen auch des weiteren Druckbehälters 1b mit einem Druckprofil mit einer zeitlich kontinuierlichen Druckänderung derart eingerichtet, dass dadurch weitere flüssige Phase aus dem weiteren Druckbehälter 1b durch die weitere Messzelle 5b förderbar ist. Die Erfasseinrichtung 11 kann zusätzlich zum Erfassen von für das Zetapotenzial indikativer Information an der weiteren Messzelle 5b während des Beaufschlagens des weiteren Druckbehälters 1b mit dem Druckprofil ausgebildet sein.

Gemäß einer Alternative ist die Erfasseinrichtung 11 ausgebildet, einen fluidischen Pfad aus dem weiteren Druckbehälter 1b und der weiteren Messzelle 5b als Referenzmesspfad für einen Messpfad aus dem Druckbehälter 1a und der Messzelle 5a einzusetzen.

Gemäß einer anderen Alternative kann ein fluidischer Pfad aus dem weiteren Druckbehälter 1b und der weiteren Messzelle 5b als zusätzlicher Messpfad zum Ermitteln von für ein Zetapotenzial an einer Grenzfläche zwischen der weiteren festen Phase und der weiteren flüssigen Phase indikativer Information ausgebildet sein.

Figur 2 zeigt den oben beschriebenen Aufbau mit einem zusätzlichen Messzweig. Der Messzweig ist aus dem weiteren Druckbehälter 1b, einem weiteren Ventil als weiteres Schaltelement 3b, der weiteren Messzelle 5b, zwei weiteren Elektroden 4b, 6b (das heißt einer weiteren Eingangselektrode und einer weiteren Ausgangselektrode) und einer weiteren Druckmesseinheit 12b gebildet. Der zusätzliche Messzweig dient zum Beispiel zur Messung einer Referenzprobe, wobei die beiden Druckbehälter 1a und 1b mit voneinander verschiedenen Lösungen gefüllt werden und somit gleiche Proben in den Messzellen 5a und 5b mit verschiedenen Lösungen durchströmt oder umströmt werden, um das unterschiedliche Adsorptionsverhalten an der Probe zu bestimmen. Dabei befinden sich die Basislösung und die Messlösung in separaten Druckbehältern 1a, 1b. Die gemessenen Drücke der beiden Druckmesseinheiten 12a und 12b werden zur Bestimmung des Zetapotenzials in die Auswerteeinheit bzw. Erfasseinrichtung 11 geliefert.

Analog wie oben beschrieben, können die beiden unterschiedlichen Lösungen in zwei separaten Vorratsbehältern 7a, 7b aufgefangen werden und mittels geeigneter Schaltung der jeweiligen Ventile (siehe Bezugszeichen 3a, 3b, 13, 15) in die jeweiligen Druckbehälter 1a oder 1b zurückbefördert werden.

Des Weiteren können zwei verschiedene Proben mit der gleichen Messlösung gemessen werden. Beispielsweise kann so eine unbehandelte Referenzprobe (Messzelle 5a) mit einer oberflächenmodifizierten Probe (Messzelle 5b) parallel gemessen und die Messergebnisse direkt verglichen werden. Hier ist es wiederum möglich, die Messlösung von Vorratsbehälter 7 (bzw. 7a, 7b) über eine geeignete Steuerung oder Regelung der jeweiligen Ventile (siehe Bezugszeichen 3a, 3b, 13, 15) zurück in den Druckbehälter 1 (bzw. 1a, 1b) zu transportieren.

Die Steuereinrichtung 10 steuert oder regelt dabei je nach gewähltem Messprinzip die Ventile (siehe Bezugszeichen 3a, 3b, 13 und 15) und die Pumpe 2. Die beschriebene Vorrichtung 20 zur pulsationsfreien Änderung der Druckdifferenz ermöglicht eine kontinuierliche Messung dieser Änderung und der zugehörigen Änderung des Strömungspotenzials oder Strömungsstroms.

**Figur 3** zeigt ein Diagramm 300, das als Beispiel eine Messung von Strömungsstrom im Druckbereich 150-800 mbar zeigt und eine Abhängigkeit zwischen der Zeit (siehe Abszisse 302), einer Druckdifferenz (siehe erste Ordinate 304) und dem Strömungsstrom (siehe zweite Ordinate 306) gemäß exemplarischen Ausführungsbeispielen der Erfindung zeigt. Figur 3 zeigt die Abnahme des Differenzdrucks und des negativen Strömungsstroms nach Druckbeaufschlagung des Druckbehälters 1. Die lineare Korrelation von Strömungsstrom und Differenzdruck (373 Messpunkte) ergibt den Strömungsstromkoeffizienten $dI_{str}/d\Delta p$=-63,3 nA/bar. Wichtig für eine präzise Zuordnung von Differenzdruck und zugehörendem Strömungspotenzial oder Strömungsstrom ist die zeitgleiche Messung dieser Parameter. Die Messung findet im Sub-Sekunden-Bereich, bevorzugt ca. alle 100 ms, statt. Der Strömungsstromkoeffizient im Beispiel in Figur 3 ergibt sich aus der linearen Regression der Abhängigkeit des Strömungsstroms vom Differenzdruck.

Bei hoher Ionenstärke verringert sich das Signal/Rausch-Verhältnis. Die zeitliche Änderung des Spannungs- oder Stromwertes unter stationären Bedingungen ($\Delta p$ = 0 bar) führt zu einer Abweichung in der Linearität des Zusammenhangs zwischen Strömungspotenzial oder Strömungsstrom und dem Differenzdruck. Die kontinuierliche Verschiebung der Basislinie, hervorgerufen durch die oben beschriebenen Effekte der Elektrodenpolarisation und elektronischer Drifterscheinungen, und deren Einfluss auf das Messsignal (Strömungspotenzial oder Strömungsstrom) werden durch aufeinanderfolgende Messungen von Spannung oder Strom bei anliegender Druckdifferenz und bei $\Delta p$ = 0 bar aufgenommen. Dabei ändert sich die anliegende Druckdifferenz während der Zyklen der Messungen von Strömungspotenzial und Strömungsstrom kontinuierlich von hohem zu niedrigem Differenzdruck.

**Figur 4** zeigt ein Diagramm 400 als Beispiel für die Messung des Strömungspotenzials während der kontinuierlichen Abnahme des Differenzdrucks und die in Serie geschaltete Messung der zeitlichen Änderung des Asymmetriepotenzials (Basislinie). Das Diagramm 400 zeigt eine Abhängigkeit zwischen der Zeit, einer Druckdifferenz und dem Strömungspotenzial (siehe zusätzliche Ordinate 402) gemäß exemplarischen Ausführungsbeispielen der Erfindung. Das Verfahren zur Korrektur der Basislinie wird dadurch verdeutlicht, dass das Strömungspotenzial (bei anliegendem Differenzdruck) bzw. das Asymmetriepotenzial (bei $\Delta p$ = 0 bar) gegen den Differenzdruck aufgetragen werden (Figur 5; Druckdaten für den stationären Zustand, d.h. $\Delta p$ = 0, sind nicht gezeigt).

**Figur 5** zeigt ein Diagramm 500, das ein Verfahren zur Korrektur einer Basislinie gemäß einem exemplarischen Ausführungsbeispiel der Erfindung dadurch verdeutlicht, dass entlang einer Ordinate 504 das Strömungspotenzial (bei anliegendem Differenzdruck) bzw. das Asymmetriepotenzial (bei $\Delta p$ = 0 bar) gegen den an der Abszisse 502 dargestellten Differenzdruck aufgetragen gezeigt ist. Figur 5 stellt somit das Strömungspotenzial als Funktion des Differenzdrucks dar. Das Strömungspotenzial bei $\Delta p$=0 bar (Druckdaten nicht angezeigt) entspricht dem Asymmetriepotenzial (Basislinie). Die Spannungswerte der Basislinie und der Druckrampe werden durch geeignete Funktionen, bevorzugt durch Polynome zweiter Ordnung, beschrieben, die für die Korrektur der Basislinie verwendet werden.

**Figur 6** zeigt ein Diagramm 600, das ein Verfahren zur Korrektur einer Basislinie gemäß einem exemplarischen Ausführungsbeispiel der Erfindung dadurch verdeutlicht, dass entlang der Ordinate 504 das Strömungspotenzial (bei anliegendem Differenzdruck) bzw. das Asymmetriepotenzial (bei $\Delta p$ = 0 bar) gegen den an der Abszisse 502 dargestellten Differenzdruck aufgetragen gezeigt ist. Figur 6 zeigt somit einen Vergleich der Druckrampe (Strömungspotenziale versus Differenzdruck) mit Basislinienkorrektur ($dU_{str}/d\Delta p$=-34,96 mV/bar) mit der Druckrampe ohne Basislinienkorrektur ($dU_{str}/d\Delta p$=-31,09 mV/bar). Der Fehler im Strömungspotenzialkoeffizient (Steigung) und damit im Zetapotenzial beträgt 12 % (wobei die Daten aus Figur 4 und Figur 5 verwendet worden sind).

Die Spannungswerte der Druckrampe (Strömungspotenzial bei anliegendem Druck) und der Basislinie (Asymmetriepotenzial bei $\Delta p$ = 0 bar) werden durch geeignete Funktionen, bevorzugt durch eine Polynomfunktion 2. Grades, beschrieben. Der zeitliche Drift der Basislinie wird vom zeitlichen Verlauf des Strömungspotenzials mit gleichzeitig abnehmender Druckdifferenz abgezogen. Ohne Basislinienkorrektur ergibt sich aus den Messwerten von Strömungspotenzial und Differenzdruck in Figur 4 und Figur 5 ein Strömungspotenzialkoeffizient $dU_{str}/d\Delta p$ = -31,085 mV/bar und ein linearer Regressionskoeffizient $r^2$ = 0,998. Mit Hilfe der hier beschriebenen Methode der Basislinienkorrektur erhält man die Werte $dU_{str}/d\Delta p$ = -34,961 mV/bar und $r^2$ = 0,9996 (Figur 6).

**Figur 7** zeigt Diagramm 700, das für das Beispiel einer Druckrampenmessung für eine leitfähige Probe in Gegenwart einer 0.001 mol/l KCl Lösung eine Abhängigkeit zwischen einer Druckdifferenz und dem Strömungspotenzial zeigt. Die Linearität des Zusammenhangs zwischen Strömungspotenzial und Differenzdruck ohne Basislinienkorrektur wird in bestimmten Szenarien als nicht ausreichend angesehen (Messpunkte in Fig. 7), in denen eine sehr hohe Messgenauigkeit gewünscht wird. Die strichlierte Linie zeigt die korrekte Steigung nach der Korrektur der Basislinie, wodurch eine weiter stark verbesserte Messgenauigkeit erhalten werden kann.

Im Weiteren werden Anwendungsbeispiele gemäß exemplarischen Ausführungsbeispielen beschrieben.

Der isoelektrische Punkt (IEP) ist definiert als jener pH Wert einer wässrigen Lösung, an dem das Zetapotenzial den Wert 0 mV annimmt. Unabhängig von der Ionenstärke der Elektrolytlösung sind die Strömungspotenzial- und Strömungsstromwerte in unmittelbarer Umgebung des IEP daher sehr niedrig. Eine zeitliche Änderung des Asymmetriepotenzials der Elektroden beeinflusst daher auch bei geringer Ionenstärke die Qualität der Messungen von Strömungspotenzial und Strömungsstrom. Bei höheren Ionenstärken tragen die Elektrodenpolarisation aufgrund der Elektrolytkonzentration und andere Drifterscheinungen zur zeitlichen Instabilität der Basislinie bei.

Die Bestimmung des IEP ist besonders kritisch bei Materialien, die aufgrund ihrer Porosität oder Quellfähigkeit in wässriger Lösung deutlich zur elektrischen Leitfähigkeit im Strömungskanal der Messzelle beitragen, sowie bei Materialien, die ihren IEP im niedrigen pH Bereich aufweisen. Als Beispiele für die Bestimmung des IEP unter diesen erschwerenden Bedingungen seien Zellulosefasern (starke Quellfähigkeit) oder der Nachweis von Sulfonsäuregruppen (IEP bei pH < 2) an entsprechend modifizierten Polymeroberflächen genannt.

Eine Messung bei hoher Ionenstärke wird möglich. Mit zunehmender Elektrolytkonzentration (Ionenstärke) erhöht sich die Polarisierbarkeit von Elektroden. Ohne Korrektur der Basislinie und ihrer zeitlichen Änderung führt eine Messung von Strömungspotenzial und Strömungsstrom bei Ionenstärken von $I > 0.1$ mol/l oft zu keinen sinnvollen Ergebnissen.

Ein Beispiel ist die Charakterisierung von Polymermembranen für die Nanofiltration und die Umkehrosmose, deren Zetapotenzial in Gegenwart hoher Ionenstärke, die der Salinität von Meerwasser entspricht ($I > 0.5$-$0.7$ mol/l), bestimmt werden soll. Ein weiteres Beispiel ist die Messung des Strömungspotenzials und Strömungsstroms an Oberflächen von Stahl- oder Titanlegierungen in Gegenwart physiologischer Pufferlösungen (> 0.15 mol/l).

Auch Adsorptionsvorgänge können ausgemessen werden. Die beschriebene Vorrichtung zur pulsationsfreien Änderung der Druckdifferenz mit gleichzeitiger Messung des Strömungspotenzials und Strömungsstroms und die ebenfalls beschriebene Methode der Basislinienkorrektur eignen sich zur Messung von Adsorptionsvorgängen in Flüssigkeit gelöster Stoffe, wie beispielsweise Tenside, Proteine und andere Polyelektrolyte, sowie in Flüssigkeit suspendierter Nanopartikel an Festkörperoberflächen unterschiedlicher Größe und Form. Die Einbringung dieser Stoffe, im Weiteren als Adsorbat bezeichnet, in die Messlösung und die Messung des Strömungspotenzials oder Strömungsstroms bei sich ändernder Konzentration von Adsorbat im Strömungskanal können zu einem weiteren Polarisationseffekt der Messelektroden führen. Die Größe dieses Effekts ist abhängig von der Art und Konzentration des Adsorbats und der Ionenstärke der Elektrolytlösung, aus der die Adsorption an der Festkörperoberfläche erfolgt. Die Messung von Adsorptionsprozessen ist im Wesentlichen bei hoher Ionenstärke erwünscht, beispielsweise in Gegenwart physiologischer Pufferlösungen ($I > 0.15$ mol/l). Komplexe Adsorbate, beispielsweise Proteine, lagern sich zumindest temporär an der Oberfläche der Messelektroden an und ändern daher das Asymmetriepotenzial (Basislinie). Eine serielle Messung von Strömungspotenzial bei anliegender Druckdifferenz (als Indikator des Adsorptionsvorgangs) und des Asymmetriepotenzials bei $\Delta p = 0$ bar (Basislinie) ist daher vorteilhaft für eine sinnvolle Analyse des Adsorptionsprozesses.

Anwendungsbeispiele sind die Analyse von Haarproben in Gegenwart von Emulsionen (z.B. Shampoo, Spülung), die Charakterisierung der Reinigungseffizienz von Spülmittel an Glasoberflächen oder der Wechselwirkung von Waschmittel und Weichspüler mit Textilgewebe, und die oben erwähnte Interaktion von Proteinen mit der Oberfläche von Biomaterialien (Metalle, Keramiken, Polymere, etc.).

Ergänzend ist darauf hinzuweisen, dass "aufweisend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Verfahren zum Ermitteln von für ein Zetapotenzial indikativer Information mittels einer Messung des Strömungsstroms oder einer Messung des Strömungspotenzials zum Charakterisieren einer Grenzfläche zwischen einer festen Phase und einer flüssigen Phase, wobei das Verfahren aufweist:

Aufnehmen der flüssigen Phase in einem Druckbehälter (1);
Aufnehmen der festen Phase in einer Messzelle (5), die stromabwärts von und in Fluidkommunikation mit dem Druckbehälter (1) bringbar angeordnet ist;
Anordnen eines Vorratsbehälters (7) stromabwärts von und in Fluidkommunikation mit der Messzelle (5);

Beaufschlagen des Druckbehälters (1) mit einem Druckprofil mit einer zeitlich kontinuierlichen Druckänderung mit einem monotonen Druckverlauf derart, dass dadurch flüssige Phase aus dem Druckbehälter (1) durch die Messzelle (5) in den Vorratsbehälter (7) förderbar ist,

wobei das Innere des Druckbehälters (1) von der Umgebung druckentkoppelt ist und sich der Druck in dem Druckbehälter (1) kontinuierlich abbaut, wenn die flüssige Phase aus dem Druckbehälter (1) durch die Messzelle (5) in den Vorratsbehälter (7) fließt, und

wobei ein Gasraum oberhalb der flüssigen Phase in dem Druckbehälter (1) mit einem komprimierten Gaspolster beaufschlagt wird, um dadurch das Druckprofil mit einer kontinuierlichen Abnahme des Drucks zu erzeugen; und

Erfassen der für das Zetapotenzial indikativen Information an der Messzelle (5) während des Beaufschlagens des Druckbehälters (1) mit dem Druckprofil,

2. Verfahren gemäß Anspruch 1, wobei

das Ermitteln der für das Zetapotenzial indikativen Information durchgeführt wird bei einer Ionenstärke von mindestens 0.001 mol/l, weiter insbesondere bei einer Ionenstärke von mindestens 0. 1 mol/l,

während eines Adsorptionsprozesses oder während eines Desorptionsprozesses, an einer metallischen Materialoberfläche, und/oder

in unmittelbarer Nähe des isoelektrischen Punktes, insbesondere bei höchstens fünf Prozent Abweichung des pH-Werts von dem isoelektrischen Punkt,

wobei insbesondere der Messzelle (5) ein Druckprofil mit einer pulsationsfreien Druckänderung bereitgestellt wird.

3. Verfahren gemäß einem der Ansprüche 1 bis 2, wobei

die für das Zetapotenzial indikative Information unter Durchführung einer Basislinienkorrektur, insbesondere einer Korrektur einer sich zeitlich ändernden Basislinie, ermittelt wird,

wobei insbesondere

die Basislinienkorrektur unter Berücksichtigung eines Asymmetriepotenzials einer Eingangselektrode (4) an einem Eingang der Messzelle (5) und einer Ausgangselektrode (6) an einem Ausgang der Messzelle (5) erfolgt, wobei insbesondere

die Basislinienkorrektur unter Berücksichtigung einer zeitlichen Änderung des Asymmetriepotenzials hinsichtlich eines Zusammenhangs zwischen einem Strömungspotenzial oder einem Strömungsstrom einerseits und einem Differenzdruck andererseits erfolgt.

4. Verfahren gemäß einem der Ansprüche 1 bis 3, wobei

ein von der festen Phase und der flüssigen Phase unabhängiges, insbesondere auf einem Asymmetriepotenzial einer Eingangselektrode (4) an einem Eingang der Messzelle (5) und einer Ausgangselektrode (6) an einem Ausgang der Messzelle (5) beruhendes, Basissignal während des Beaufschlagens des Druckbehäiters (1) mit dem Druckprofil ermittelt wird, und/oder wobei

während der Druckänderung alternierend zwischen einem eine Fluidverbindung zwischen dem Druckbehälter (1) und der Messzelle (5) zulassenden Betriebsmodus und einem eine Fluidverbindung zwischen dem Druckbehälter (1) und der Messzelle (5) unterbindenden Betriebsmodus gewechselt wird, um in dem die Fluidverbindung unterbindenden Betriebsmodus, insbesondere mehrere Male, ein Basisliniensignal zu erfassen und damit ein in dem die Fluidverbindung zulassenden Betriebsmodus erfasstes Messsignal zu korrigieren.

5. Vorrichtung (20) zum Ermitteln von für ein Zetapotenzial indikativer Information mittels einer Messung des Strömungsstroms oder einer Messung des Strömungspotenzials zum Charakterisieren einer Grenzfläche zwischen einer festen Phase und einer flüssigen Phase, wobei die Vorrichtung (20) aufweist:

einen Druckbehälter (1), in dem die flüssige Phase aufnehmbar ist;

eine Messzelle (5), die stromabwärts von und in Fluidkommunikation mit dem Druckbehälter (1) bringbar angeordnet ist und in der die feste Phase aufnehmbar ist;

einen Vorratsbehälter (7), der stromabwärts von und in Fluidkommunikation mit der Messzelle (5) angeordnet ist;

eine Druckbeaufschlagungseinrichtung (19), die zum Beaufschlagen des Druckbehälters (1) mit einem Druckprofil mit einer zeitlich kontinuierlichen Druckänderung mit einem monotonen Druckverlauf derart eingerichtet ist, dass dadurch flüssige Phase aus dem Druckbehälter (1) durch die Messzelle (5) in den Vorratsbehälter (7) förderbar ist,

wobei das Innere des Druckbehälters (1) von der Umgebung druckentkoppelbar ist und sich der Druck in dem Druckbehälter (1) kontinuierlich abbaut, wenn die flüssige Phase aus dem Druckbehälter (1) durch die Messzelle (5) in den Vorratsbehälter (7) fließt,

wobei die Druckbeaufschlagungseinrichtung (19) eingerichtet ist, einen Gasraum oberhalb der flüssigen Phase in dem Druckbehälter (1) mit einem komprimierten Gaspolster zu beaufschlagen, um dadurch das Druckprofil mit einer kontinuierlichen Abnahme des Drucks zu erzeugen; und

eine Erfasseinrichtung (11) zum Erfassen der für das Zetapotenzial indikativen Information an der Messzelle (5) während des Beaufschlagens des Druckbehälters (1) mit dem Druckprofil.

6. Vorrichtung (20) gemäß Anspruch 5, wobei
die Druckbeaufschlagungseinrichtung (19) ausgebildet ist, der Messzelle (5) ein Druckprofil mit einer pulsationsfreien Druckänderung bereitzustellen.

7. Vorrichtung (20) gemäß einem der Ansprüche 5 bis 6, wobei

die Druckbeaufschlagungseinrichtung (19) eine Druckerzeugungseinheit (2, 14) zum Erzeugen von Druck in dem Druckbehälter (1) und ein Schaltelement (3) aufweist, so dass mittels Schaltens des Schaltelements (3) zum fluidischen Koppeln des Druckbehälters (1) mit der Messzelle (5) die Messzelle (5) mit der flüssigen Phase entsprechend dem Druckprofil beaufschlagbar ist, wobei insbesondere

die Druckerzeugungseinheit aus einer Gruppe ausgewählt ist, die besteht aus einer Pumpe (2), insbesondere einer Membranpumpe, und/oder einer Gasdruckversorgung (14), insbesondere einer Gasflasche, und/oder das Schaltelement (3) zwischen dem Druckbehälter (1) und der Messzelle (5) angeordnet ist.

8. Vorrichtung (20) gemäß einem der Ansprüche 5 bis 7, aufweisend eine Druckmesseinheit (12) zum Erfassen eines Drucks, insbesondere stromaufwärts von oder an dem Druckbehälter (1).

9. Vorrichtung (20) gemäß Anspruch 8, sofern rückbezogen auf Anspruch 7, wobei
das Schaltelement (3) derart schaltbar ist, dass es bei mittels der Druckmesseinheit (12) festgestellter Überschreitung eines vorgebbaren Druckschwellwerts die Fluidverbindung zwischen dem Druckbehälter (1) und der Messzelle (5) aktiviert.

10. Vorrichtung (20) gemäß einem der Ansprüche 5 bis 9,

wobei der Vorratsbehälter (7) überdruckfrei konfiguriert ist, insbesondere dessen Inneres auf Atmosphärendruck befindlich ist, und/oder

wobei die Erfasseinrichtung (11) zum Erfassen der für das Zetapotenzial indikativen Information basierend auf einer elektrischen Strommessung und/oder basierend auf einer elektrischen Spannungsmessung an der Messzelle (5) eingerichtet ist, und/oder

wobei eine Eingangselektrode (4) an einem Eingang der Messzelle (5) und eine Ausgangselektrode (6) an einem Ausgang der Messzelle (5), wobei die Erfasseinrichtung (11) zum Erfassen der für das Zetapotenzial indikativen Information zwischen der Eingangselektrode (4) und der Ausgangselektrode (6) ausgebildet ist, wobei insbesondere

die Eingangselektrode (4) zum Wechselwirken zumindest mit der flüssigen Phase an dem Eingang der Messzelle (5) und die Ausgangselektrode (6) zum Wechselwirken zumindest mit der flüssigen Phase an dem Ausgang der Messzelle (5) angeordnet ist, wobei insbesondere

die Erfasseinrichtung (11) ausgebildet ist, mittels der Eingangselektrode (4) und mittels der Ausgangselektrode (6) eine für einen elektrischen Widerstand der festen Phase und der flüssigen Phase in der Messzelle (5) indikative Information zu erfassen, wobei insbesondere

die Erfasseinrichtung (11) ausgebildet ist, basierend auf der für den elektrischen Widerstand indikativen Information zumindest eine Information abzuleiten, die aus einer Gruppe ausgewählt ist, die besteht aus Information hinsichtlich Gasblasen in der Messzelle (5), und einem Beitrag der festen Phase zur elektrischen Gesamtleitfähigkeit.

11. Vorrichtung (20) gemäß einem der Ansprüche 5 bis 10, aufweisend
einen Flüssigphasenrückfördermechanismus zum Rückfördern von flüssiger Phase von dem Vorratsbehälter (7) in den Druckbehälter (1).

12. Vorrichtung (20) gemäß Anspruch 11, sofern rückbezogen auf Anspruch 7, wobei

das Schaltelement (3) in einem fluidischen Pfad angeordnet ist, in dem mittels des Flüssigphasenrückfördermechanismus flüssige Phase von dem Vorratsbehälter (7) zu dem Druckbehälter (1) rückförderbar ist.

**13.** Vorrichtung (20) gemäß einem der Ansprüche 5 bis 12, ferner aufweisend:

einen weiteren Druckbehälter (1b), in dem weitere flüssige Phase aufnehmbar ist;
eine weitere Messzelle (5b), die stromabwärts von und in Fluidkommunikation mit dem weiteren Druckbehälter (1b) bringbar angeordnet ist und in der weitere feste Phase aufnehmbar ist;
wobei die Druckbeschlageinrichtung (19) zum Beaufschlagen des weiteren Druckbehälters (1b) mit einem Druckprofil mit einer zeitlich kontinuierlichen Druckänderung derart eingerichtet ist, dass dadurch weitere flüssige Phase aus dem weiteren Druckbehälter (1b) durch die weitere Messzelle (5b) förderbar ist; wobei die Erfasseinrichtung (11) zum Erfassen von für das Zetapotenzial indikativer Information an der weiteren Messzelle (5b) während des Beaufschlagens des weiteren Druckbehälters (1b) mit dem Druckprofil ausgebildet ist, wobei insbesondere
die Erfasseinrichtung (11) ausgebildet ist, einen fluidischen Pfad aus dem weiterem Druckbehälter (1b) und der weiteren Messzelle (5b) als Referenzmesspfad für einen Messpfad aus dem Druckbehälter (1a) und der Messzelle (5a) einzusetzen, oder
ein fluidischer Pfad aus dem weiteren Druckbehälter (1b) und der weiteren Messzelle (5b) als zusätzlicher Messpfad zum Ermitteln von für ein Zetapotenzial indikativer Information zum Charakterisieren einer Grenzfläche zwischen der weiteren festen Phase und der weiteren flüssigen Phase ausgebildet ist.

**14.** Vorrichtung (20) gemäß einem der Ansprüche 5 bis 13, wobei

die Erfasseinrichtung (11) zum Erfassen der für das Zetapotenzial indikativen Information unter Durchführung einer Basislinienkorrektur, insbesondere einer Korrektur einer sich zeitlich ändernden Basislinie, eingerichtet ist, wobei insbesondere
die Basislinienkorrektur unter Berücksichtigung eines Asymmetriepotenzials einer Eingangselektrode (4) an einem Eingang der Messzelle (5) und einer Ausgangselektrode (6) an einem Ausgang der Messzelle (5) erfolgt, wobei insbesondere
die Basislinienkorrektur unter Berücksichtigung einer zeitlichen Änderung des Asymmetriepotenzials hinsichtlich eines Zusammenhangs zwischen dem Strömungspotenzial oder dem Strömungsstrom einerseits und einem Differenzdruck andererseits erfolgt.

**15.** Vorrichtung (20) gemäß einem der Ansprüche 5 bis 14, wobei

die Erfasseinrichtung (11) zum Erfassen eines von der festen Phase und der flüssigen Phase unabhängigen, insbesondere auf einem Asymmetriepotenzial einer Eingangselektrode (4) an einem Eingang der Messzelle (5) und einer Ausgangselektrode (6) an einem Ausgang der Messzelle (5) beruhenden, Basissignals während des Beaufschlagens des Druckbehälters (1) mit dem Druckprofil eingerichtet ist, und/oder wobei
die Druckbeaufschlagungseinrichtung (19) zum Beaufschlagen des Druckbehälters (1) mit einem Druckprofil derart eingerichtet ist, dass eine Druckdifferenz zwischen einem Anfangsdruckwert an dem Druckbehälter (1) und einem Anfangsdruckwert an dem Vorratsbehälter (7) größer als eine Atmosphäre ist, insbesondere größer als 2 bar ist, weiter insbesondere zwischen 5 bar und 10 bar ist.

## Claims

**1.** Method of determining an information which is indicative for a zeta potential by a measurement of the flow current or a measurement of the flow potential for characterizing a boundary surface between a solid phase and a liquid phase, wherein the method comprises:

receiving the liquid phase in a pressure container (1);
receiving the solid phase in a measuring cell (5) which is arranged downstream of the pressure container (1) and can be brought in fluid communication with the pressure container (1);
arranging a storage container (7) downstream and in fluid communication with the measuring cell (5);
pressurizing the pressure container (1) with a pressure profile with a pressure change which is continuous over time with a monotonous pressure course, such that thereby the liquid phase is conveyable from the pressure container (1) through the measuring cell (5) in the storage container (7),

wherein the interior of the pressure container (1) is pressure-decoupled from the environment and the pressure in the pressure container (1) continuously decreases, when the liquid phase flows from the pressure container (1) through the measuring cell (5) in the storage container (7), and
wherein a gas space above the liquid phase and the pressure container (1) is pressurized with a compressed gas cushion, to thereby generate the pressure profile with a continuous decrease of pression; and
capturing the information which is indicative for the zeta potential at the measuring cell (5) during pressurizing the pressure container (1) with the pressure profile.

2. Method according to claim 1, wherein
determining the information which is indicative for the zeta potential is performed

at an ionic strength of at least 0.001 mol/l, further in particular at an ionic strength of at least 0.1 mol/l,
during an adsorption process or during a desorption process,
at a metallic material surface, and/or
in close vicinity to the isoelectric point, in particular with maximum five percent deviation of the pH value from the isoelectric point,
wherein in particular a pressure profile with a pulsation-free pressure change is provided to the measuring cell (5).

3. Method according to one of the claims 1 to 2, wherein
the information which is indicative for the zeta potential is determined under performance of a baseline correction, in particular a correction of a baseline which changes over time,
wherein in particular

the baseline correction is performed under consideration of an asymmetry potential of an inlet electrode (4) at an inlet of the measuring cell (5) and an outlet electrode (6) at an outlet of the measuring cell (5), wherein in particular
the baseline correction is performed under consideration of a change of the asymmetry potential over time with respect to a connection between a flow potential or a flow current on the one hand and a pressure difference on the other hand.

4. Method according to one of the claims 1 to 3, wherein

a base signal which is independent from the solid phase and the liquid phase, which is in particular based on an asymmetry potential of an inlet electrode (4) at an inlet of the measuring cell (5) and an outlet electrode (6) at an outlet of the measuring cell (5) is determined during pressurizing the pressure container (1) with the pressure profile, and/or wherein
during the pressure change, it is alternatingly changed between an operation mode which allows a fluid connection between the pressure container (1) and the measuring cell (5) and an operation mode which prevents a fluid connection between the pressure container (1) and the measuring cell (5), to capture, in particular multiple times, a baseline signal in the operation mode which prevents the fluid connection and to correct a measuring signal with it which is captured in the operation mode which allows the fluid connection.

5. Device (20) for determining an information which is indicative for a zeta potential by a measurement of the flow current or a measurement of the flow potential for characterizing a boundary surface between a solid phase and a liquid phase, wherein the device (20) comprises:

a pressure container (1) in which the liquid phase is receivable;
a measuring cell (5) which is arranged downstream of the pressure container (1) and can be brought in fluid communication with the pressure container (1), and in which the solid phase is receivable;
a storage container (7) which is arranged downstream of and in fluid communication with the measuring cell (5);
a pressurizing unit (19) which is configured for pressurizing the pressure container (1) with a pressure profile with a pressure change which is continuous over time with a monotonous pressure course, such that thereby the liquid phase is conveyable from the pressure container (1) through the measuring cell (5) in the storage container (7),
wherein the interior of the pressure container (1) is pressure-decouplable from the environment, and the pressure in the pressure container (1) continuously decreases, when the liquid phase flows from the pressure container (1) through the measuring cell (5) in the storage container (7),
wherein the pressurizing unit (19) is configured to pressurize a gas space above the liquid phase in the pressure container (1) with a compressed gas cushion, to thereby generate the pressure profile with a continuous decrease of the pressure; and
a capturing unit (11) for capturing the information which is indicative for the zeta potential at the measuring cell (5) during pressurizing the pressure container (1) with the pressure profile.

6. Device (20) according to claim 5, wherein
the pressurizing unit (19) is configured to provide a pressure profile with a pulsation-free pressure change to the measuring cell (5).

7. Device (20) according to one of the claims 5 to 6, wherein

the pressurizing unit (19) comprises a pressure generating unit (2, 14) for generating pressure in the pressure container (1) and a switching element (3), so that, by switching the switching element (3) for fluidically coupling the pressure container (1) with the measuring cell (5), the measuring cell (5) is pressurizable with the liquid phase in a manner corresponding to the pressure profile, wherein in particular
the pressure generating unit is selected from a group which is consisting of a pump (2), in particular a membrane pump, and/or a gas pressure supply (14), in particular a gas bottle, and/or
the switching element (3) is arranged between the pressure container (1) and the measuring cell (5).

8. Device (20) according to one of the claims 5 to 7, comprising
a pressure measuring unit (12) for capturing a pressure, in particular upstream of or at the pressure container (1).

9. Device (20) according to claim 8, if referencing back to claim 7, wherein
the switching element (3) is switchable such that, in case of an exceedance of a pregivable pressure threshold value, which is determined by the pressure measuring unit (12), it activates the fluid connection between the pressure container (1) and the measuring cell (5).

10. Device (20) according to one of the claims 5 to 9,

wherein the storage container (7) is configured in an overpressure-free manner, in particular its interior is at atmospheric pressure, and/or
wherein the capturing unit (11) is configured for capturing the information which is indicative for the zeta potential based on an electric current measurement and/or based on an electric voltage measurement at the measuring cell (5), and/or
wherein an inlet electrode (4) is at an inlet of the measuring cell (5) and an outlet electrode (6) is at an outlet of the measuring cell (5), wherein the capturing unit (11) for capturing the information which is indicative for the zeta potential is formed between the inlet electrode (4) and the outlet electrode (6), wherein in particular
the inlet electrode (4) is arranged for interacting at least with the liquid phase at the inlet of the measuring cell (5), and the outlet electrode (6) is arranged for interacting at least with the liquid phase at the outlet of the measuring cell (5), wherein in particular
the capturing unit (11) is configured to capture an information which is indicative for an electric resistance of the solid phase and the liquid phase in the measuring cell (5) by the inlet electrode (4) and by the outlet electrode (6), wherein in particular
the capturing unit (11) is configured to derive at least one information based on the information which is indicative for the electrical resistance, which is selected from a group which is consisting of an information with respect to gas bubbles in the measuring cell (5), and a contribution of the solid phase to the total electrical conductivity.

11. Device (20) according to one of the claims 5 to 10, comprising
a liquid phase re-conveying mechanism for re-conveying the liquid phase from the storage container (7) in the pressure container (1).

12. Device (20) according to claim 11, if referencing back to claim 7, wherein
the switching element (3) is arranged in a fluidic path, in which, by the liquid phase re-conveying mechanism, the liquid phase is re-conveyable from the storage container (7) to the pressure container (1).

13. Device (20) according to one of the claims 5 to 12, further comprising:

a further pressure container (1b) in which further liquid phase is receivable;

a further measuring cell (5b) which is arranged downstream of the further pressure container (1b) and can be brought in fluid communication with the further pressure container (1b), and in which further solid phase is receivable;

wherein the pressurizing unit (19) is configured for pressurizing the further pressure container (1b) with a pressure profile with a pressure change which is continuous over time, such that thereby the further liquid phase is conveyable from the further pressure container (1b) through the further measuring cell (5b);

wherein the capturing unit (11) is configured for capturing an information which is indicative for the zeta potential at the further measuring cell (5b) during pressurizing the further pressure container (1b) with a pressure profile,

wherein in particular

the capturing unit (11) is configured to use a fluidic path from the further pressure container (1b) and the further measuring cell (5b) as a reference measuring path for a measuring path from the pressure container (1a) and the measuring cell (5a), or

a fluidic path from the further pressure container (1b) and the further measuring cell (5b) is used as an additional measuring path for determining an information which is indicative for a zeta potential, for characterizing a boundary surface between the further solid phase and the further liquid phase.

**14.** Device (20) according to one of the claims 5 to 13, wherein

the capturing unit (11) is configured for capturing the information which is indicative for the zeta potential under performance of a baseline correction, in particular a correction of a baseline which changes over time, wherein in particular

the baseline correction is performed under consideration of an asymmetry potential of an inlet electrode (4) at an inlet of the measuring cell (5) and an outlet electrode (6) at an outlet of the measuring cell (5), wherein in particular

the baseline correction is performed under consideration of a change over time of the asymmetry potential with respect to a connection between the flow potential or the flow current on the one hand and a pressure difference on the other hand.

**15.** Device (20) according to one of the claims 5 to 14, wherein

the capturing unit (11) is configured for capturing a base signal which is independent from the solid phase and the liquid phase, which is in particular based on an asymmetry potential of an inlet electrode (4) at an inlet of the measuring cell (5) and an outlet electrode (6) at an outlet of the measuring cell (5), during pressurizing the pressure container (1) with a pressure profile, and/or wherein

the pressurizing unit (19) is configured for pressurizing the pressure container (1) with a pressure profile, such that a pressure difference between an initial pressure value at the pressure container (1) and an initial pressure value at the storage container (7) is larger than 1 atm, is in particular larger than 2 bar, further in particular between 5 bar and 10 bar.

**Revendications**

**1.** Procédé pour la détermination d'information indiquant un potentiel zêta au moyen d'une mesure du courant d'écoulement ou d'une mesure du potentiel d'écoulement pour la caractérisation d'une surface limite entre une phase solide et une phase liquide, **caractérisé en ce que** le procédé présente :

la réception de la phase liquide dans un récipient sous pression (1) ;

la réception de la phase solide dans une cellule de mesure (5), qui est disposée de manière à pouvoir être amenée en aval de et en communication fluidique avec le récipient sous pression (1) ;

la disposition d'un récipient de stockage (7) en aval de et en communication fluidique avec la cellule de mesure (5) ;

la sollicitation du récipient sous pression (1) avec un profil de pression avec un changement de pression continu dans le temps avec une variation de pression monotone, de telle sorte que la phase liquide peut ainsi être transportée hors du récipient sous pression (1) à travers la cellule de mesure (5) dans le récipient de stockage (7), dans lequel l'intérieur du récipient sous pression (1) est découplé en pression de l'environnement et la pression dans le récipient sous pression (1) diminue de manière continue, lorsque la phase liquide s'écoule hors du récipient sous pression (1) à travers la cellule de mesure (5) dans le récipient de stockage (7), et
dans lequel un espace de gaz au-dessus de la phase liquide dans le récipient sous pression (1) est sollicité avec un coussin de gaz comprimé, afin de produire ainsi le profil de pression avec une réduction continue de la pression ; et
l'acquisition de l'information indiquant le potentiel zêta sur la cellule de mesure (5) pendant la sollicitation du récipient sous pression (1) avec le profil de pression.

2. Procédé selon la revendication 1, **caractérisé en ce que**

la détermination de l'information indiquant le potentiel zêta est mise en œuvre pour une force ionique d'au moins 0,001 mol/l, plus particulièrement pour une force ionique d'au moins 0,1 mol/l,
pendant un processus d'adsorption ou pendant un processus de désorption, sur une surface en matériau métallique, et/ou
à proximité immédiate du point isoélectrique, en particulier pour au maximum cinq pour cent d'écart du pH par rapport au point isoélectrique,
dans lequel en particulier un profil de pression avec un changement de pression exempt de pulsation est fourni à la cellule de mesure (5).

3. Procédé selon l'une quelconque des revendications 1 à 2, **caractérisé en ce que**

l'information indiquant le potentiel zêta est déterminée par mise en œuvre d'une correction de ligne de base, en particulier d'une correction d'une ligne de base changeant dans le temps,
dans lequel en particulier
la correction de ligne de base s'effectue avec prise en compte d'un potentiel d'asymétrie d'une électrode d'entrée (4) à une entrée de la cellule de mesure (5) et d'une électrode de sortie (6) à une sortie de la cellule de mesure (5), dans lequel en particulier
la correction de ligne de base s'effectue avec prise en compte d'un changement temporel du potentiel d'asymétrie en ce qui concerne une relation entre un potentiel d'écoulement ou un courant d'écoulement d'une part et une pression différentielle d'autre part.

4. Procédé selon l'une quelconque des revendications 1 à 3, **caractérisé en ce que**

un signal de base indépendant de la phase solide et de la phase liquide, reposant en particulier sur un potentiel d'asymétrie d'une électrode d'entrée (4) à une entrée de la cellule de mesure (5) et d'une électrode de sortie (6) à une sortie de la cellule de mesure (5), est déterminé pendant la sollicitation du récipient sous pression (1) avec le profil de pression, et/ou dans lequel
pendant le changement de pression on bascule en alternance entre un mode de fonctionnement autorisant une liaison fluidique entre le récipient sous pression (1) et la cellule de mesure (5) et un mode de fonctionnement empêchant une liaison fluidique entre le récipient sous pression (1) et la cellule de mesure (5), afin d'acquérir dans le mode de fonctionnement empêchant la liaison fluidique, en particulier plusieurs fois, un signal de ligne de base et de corriger ainsi un signal de mesure acquis dans le mode de fonctionnement autorisant la liaison fluidique.

5. Dispositif (20) pour la détermination d'information indiquant un potentiel zêta au moyen d'une mesure du courant d'écoulement ou d'une mesure du potentiel d'écoulement pour la caractérisation d'une surface limite entre une phase solide et une phase liquide, **caractérisé en ce que** le dispositif (20) présente :

un récipient sous pression (1), dans lequel la phase liquide peut être reçue ;
une cellule de mesure (5), qui est disposée de manière à pouvoir être amenée en aval de et en communication fluidique avec le récipient sous pression (1) et dans laquelle la phase solide peut être reçue ;
un récipient de stockage (7), qui est disposé en aval de et en communication fluidique avec la cellule de mesure (5) ;
un système de sollicitation en pression (19), qui est conçu pour solliciter le récipient sous pression (1) avec un profil de pression avec un changement de pression continu dans le temps avec une variation de pression monotone de telle sorte que la phase liquide peut être ainsi transportée hors du récipient sous pression (1) à travers la cellule de mesure (5) dans le récipient de stockage (7),
dans lequel l'intérieur du récipient sous pression (1) peut être découplé en pression de l'environnement et la pression dans le récipient sous pression (1) diminue de manière continue, lorsque la phase liquide circule hors du récipient sous pression (1) à travers la cellule de mesure (5) dans le récipient de stockage (7), dans lequel le système de sollicitation en pression (19) est conçu pour solliciter un espace de gaz au-dessus de la phase liquide dans le récipient sous pression (1) avec un coussin de gaz comprimé, afin de produire ainsi le profil de pression avec une réduction continue de la pression ; et un système d'acquisition (11) pour l'acquisition de l'information indiquant le potentiel zêta sur la cellule de mesure (5) pendant la sollicitation du récipient sous pression (1) avec le profil de pression.

6. Dispositif (20) selon la revendication 5, **caractérisé en ce que** le système de sollicitation en pression (19) est réalisé pour fournir à la cellule de mesure (5) un profil de pression avec un changement de pression exempt de pulsation.

7. Dispositif (20) selon l'une quelconque des revendications 5 à 6, **caractérisé en ce que**

le système de sollicitation en pression (19) présente une unité de génération de pression (2, 14) pour la génération de pression dans le récipient sous pression (1) et un élément de commutation (3), de sorte que la cellule de mesure (5) peut être sollicitée avec la phase liquide de manière correspondante au profil de pression au moyen de la commutation de l'élément de commutation (3) pour le couplage fluidique du récipient sous pression (1) à la cellule de mesure (5), dans lequel en particulier l'unité de génération de pression est choisie dans un groupe qui est constitué d'une pompe (2), en particulier d'une pompe à membrane, et/ou d'une alimentation en gaz comprimé (14), en particulier d'une bouteille de gaz, et/ou l'élément de commutation (3) est disposé entre le récipient sous pression (1) et la cellule de mesure (5).

8. Dispositif (20) selon l'une quelconque des revendications 5 à 7, présentant une unité de mesure de pression (12) pour l'acquisition d'une pression, en particulier en amont de ou sur le récipient sous pression (1) .

9. Dispositif (20) selon la revendication 8, dans la mesure où elle est rattachée à la revendication 7, **caractérisé en ce que** l'élément de commutation (3) peut être commuté de telle sorte que lorsqu'un dépassement d'une valeur seuil de pression pouvant être prédéfinie est établi au moyen de l'unité de mesure de pression (12), la liaison fluidique entre le récipient sous pression (1) et la cellule de mesure (5) est activée.

10. Dispositif (20) selon l'une quelconque des revendications 5 à 9,

**caractérisé en ce que** le récipient de stockage (7) est configuré de manière exempte de surpression, en particulier l'intérieur de celui-ci se trouve à la pression atmosphérique, et/ou dans lequel le système d'acquisition (11) est conçu pour l'acquisition de l'information indiquant le potentiel zêta sur la base d'une mesure de courant électrique et/ou sur la base d'une mesure de tension électrique sur la cellule de mesure (5), et/ou dans lequel une électrode d'entrée (4) à une entrée de la cellule de mesure (5) et une électrode de sortie (6) à une sortie de la cellule de mesure (5), dans lequel le système d'acquisition (11) est réalisé pour l'acquisition de l'information indiquant le potentiel zêta entre l'électrode d'entrée (4) et l'électrode de sortie (6), dans lequel en particulier l'électrode d'entrée (4) est disposée pour l'interaction au moins avec la phase liquide à l'entrée de la cellule de mesure (5) et l'électrode de sortie (6) pour l'interaction au moins avec la phase liquide à la sortie de la cellule de mesure (5), dans lequel en particulier le système d'acquisition (11) est réalisé pour acquérir au moyen de l'électrode d'entrée (4) et au moyen de l'électrode de sortie (6) une information indiquant une résistance électrique de la phase solide et de la phase liquide dans la cellule de mesure (5), dans lequel en particulier le système d'acquisition (11) est réalisé pour déduire au moins une information sur la base de l'information indiquant la résistance électrique, qui est choisie dans un groupe qui est constitué d'une information concernant des bulles de gaz dans la cellule de mesure (5), et d'une contribution de la phase solide à la conductivité électrique totale.

11. Dispositif (20) selon l'une quelconque des revendications 5 à 10, présentant un mécanisme de renvoi de phase liquide pour le renvoi de la phase liquide à partir du récipient de stockage (7) dans le récipient sous pression (1).

12. Dispositif (20) selon la revendication 11, dans la mesure où elle est rattachée à la revendication 7, **caractérisé en ce que**

l'élément de commutation (3) est disposé dans une voie fluidique, dans laquelle la phase liquide peut être renvoyée à partir du récipient de stockage (7) vers le récipient sous pression (1) au moyen du mécanisme de renvoi de phase liquide.

**13.** Dispositif (20) selon l'une quelconque des revendications 5 à 12, présentant en outre :

un autre récipient sous pression (1b), dans lequel une autre phase liquide peut être reçue ;
une autre cellule de mesure (5b), qui est disposée de manière à pouvoir être amenée en aval de et en communication fluidique avec l'autre récipient sous pression (1b) et peut être reçue dans l'autre phase solide ;
**caractérisé en ce que** le système de sollicitation en pression (19) est conçu pour la sollicitation de l'autre récipient sous pression (1b) avec un profil de pression avec un changement de pression continu dans le temps, de telle sorte qu'ainsi l'autre phase liquide peut être transportée à partir de l'autre récipient sous pression (1b) à travers l'autre cellule de mesure (5b) ;
dans lequel le système d'acquisition (11) est réalisé pour l'acquisition d'information indiquant le potentiel zêta sur l'autre cellule de mesure (5b) pendant la sollicitation de l'autre récipient sous pression (1b) avec le profil de pression, dans lequel en particulier
le système d'acquisition (11) est réalisé pour utiliser une voie fluidique composée de l'autre récipient sous pression (1b) et de l'autre cellule de mesure (5b) comme voie de mesure de référence pour une voie de mesure composée du récipient sous pression (1a) et de la cellule de mesure (5a), ou
une voie fluidique composée de l'autre récipient sous pression (1b) et de l'autre cellule de mesure (5b) est réalisée comme voie de mesure supplémentaire pour la détermination d'information indiquant un potentiel zêta pour la caractérisation d'une surface limite entre l'autre phase solide et l'autre phase liquide.

**14.** Dispositif (20) selon l'une quelconque des revendications 5 à 13, **caractérisé en ce que**

le système d'acquisition (11) est conçu pour l'acquisition de l'information indiquant le potentiel zêta avec mise en œuvre d'une correction de ligne de base, en particulier d'une correction d'une ligne de base changeant dans le temps, dans lequel en particulier
la correction de ligne de base s'effectue avec prise en compte d'un potentiel d'asymétrie d'une électrode d'entrée (4) à une entrée de la cellule de mesure (5) et d'une électrode de sortie (6) à une sortie de la cellule de mesure (5), dans lequel en particulier
la correction de ligne de base s'effectue avec prise en compte d'un changement temporel du potentiel d'asymétrie en ce qui concerne une liaison entre le potentiel d'écoulement ou le courant d'écoulement d'une part et une pression différentielle d'autre part.

**15.** Dispositif (20) selon l'une quelconque des revendications 5 à 14, **caractérisé en ce que**

le système d'acquisition (11) est conçu pour l'acquisition d'un signal de base indépendant de la phase solide et de la phase liquide, reposant en particulier sur un potentiel d'asymétrie d'une électrode d'entrée (4) à une entrée de la cellule de mesure (5) et d'une électrode de sortie (6) à une sortie de la cellule de mesure (5) pendant la sollicitation du récipient sous pression (1) avec le profil de pression, et/ou dans lequel
le système de sollicitation en pression (19) est conçu pour la sollicitation du récipient sous pression (1) avec un profil de pression de telle sorte qu'une pression différentielle entre une valeur de pression initiale sur le récipient sous pression (1) et une valeur de pression initiale sur le récipient de stockage (7) est supérieure à une atmosphère, est en particulier supérieure à 2 bar, plus particulièrement est comprise entre 5 bar et 10 bar.

Fig. 1

EP 2 944 952 B1

Fig. 2

Fig. 3

EP 2 944 952 B1

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 8600707 A
- JP H02216443 B
- WO 2007065825 A
- DD 258470
- DE 10154790
- JP 62047545 B
- DE 20209563
- JP 11190711 B
- JP 04050758 B
- US 6023661 A

**In der Beschreibung aufgeführte Nicht-Patentliteratur**

- **PU et al.** Label-free detection of heparin, streptavidin, and other probes by pulsed streaming potenzials in plastic microfluidic channels. *Anal Chem,* 01. September 2008, vol. 80 (17), 6532-6
- **LUNA-VERA.** Adsorption kinetics of proteins in plastic microfluidic channels: Real-time monitoring of lysozyme adsorption by pulsed streaming potenzials. *Biosensors and Bioelectronics,* 2010, vol. 25, 1539-1543
- **VINOGRADOV J ; JAAFAR M Z ; JACKSON M D.** Measurement of streaming potenzial coupling coefficient in sandstones saturated with natural and artificial brines at high salinity. *J Geophys Res,* 2010, vol. 115 (B1), 2204